# EUROPÄISCHE PATENTANMELDUNG

(11) **EP 0 589 313 A1**
(43) Veröffentlichungstag der Anmeldung: **30.03.1994**
(21) Anmeldenummer: 93114620.3
(22) Anmeldetag: 11.09.1993
(51) Int. Cl.: C07C 233/65, C07D 213/82, C07D 327/06, C07D 333/38, C07D 231/14, C07D 277/56, C07D 335/02, C07D 309/28, C07D 307/68, A01N 43/40, A01N 43/50, A01N 43/78, A01N 43/84, A01N 37/22

(54) **Cyclohex(en)ylcarbonsäureamide, Verfahren zu ihrer Herstellung und sie enthaltende Mittel zur Bekämpfung von Schadpilzen**

(30) Priorität: 21.09.1992 DE 4231519
(71) Anmelder: BASF Aktiengesellschaft, D-67063 Ludwigshafen (DE)
(72) Erfinder: Eicken, Karl, Dr., D-6706 Wachenheim (DE); Ammermann, Eberhard, Dr., D-6148 Heppenheim (DE); Lorenz, Gisela, Dr., D-6730 Neustadt (DE)

(57) **Zusammenfassung**

N-Cyclohex(en)ylcarbonsäureamide der Formel I 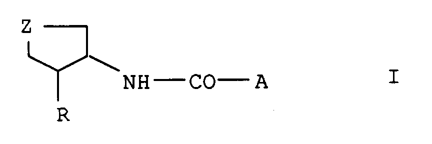
in der die Substituenten die folgende Bedeutung haben:
- R: ggf. subst. Alkyl, Alkoxy, Alkenyl, Alkenyloxy, Alkinyl, Alkinyloxy, Cycloalkyl, Cycloalkenyl, Cycloalkyloxy, Cycloalkenyloxy, Phenyl oder Benzyl;
- Z: CH₂CH₂ oder CH=CH;
- A: einer der Reste A1 bis A7: 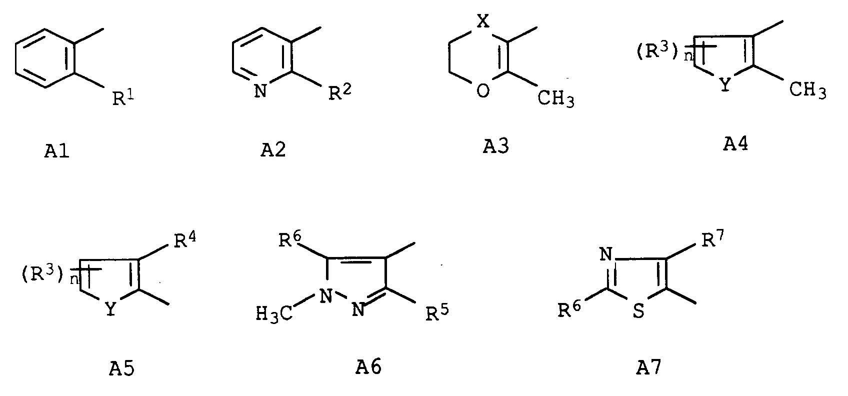
mit
- X: -CH₂-, -S-, -SO- oder -SO₂-;
- Y: -O- oder -S-;
- R¹, R², R⁴, R₅ und R⁷: Halogen, Alkyl oder Halogenalkyl;
- R³ und R⁶: Wasserstoff, Halogen oder Alkyl;
- n: 1 oder 2;
Verfahren zu ihrer Herstellung, sowie sie enthaltende Mittel und deren Verwendung zur Bekämpfung von Schadpilzen.

## Beschreibung

Die vorliegende Erfindung betrifft N-Cyclohex(en)ylcarbonsäureamide der Formel I 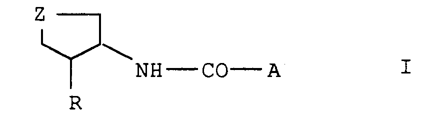
in der die Substituenten die folgende Bedeutung haben:
- R: C₂-C₁₂-Alkyl, C₂-C₁₂-Alkoxy, C₃-C₁₂-Alkenyl, C₃-C₁₂-Alkenyloxy, C₃-C₆-Alkinyl, C₃-C₆-Alkinyloxy, wobei diese Gruppen partiell oder vollständig halogeniert sein können; C₃-C₇-Cycloalkyl, C₄-C₇-Cycloalkenyl, C₃-C₇-Cycloalkyloxy oder C₄-C₇-Cycloalkenyloxy, wobei diese Ringe ein bis drei C₁-C₄-Alkylgruppen tragen können; Phenyl oder Benzyl, wobei die Phenylringe jeweils ein bis fünf Halogenatome und/oder ein bis drei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogenalkylthio;
- Z: CH₂CH₂ oder CH=CH;
- A: ein cyclischer Rest aus der Gruppe der Formeln A1 bis A7 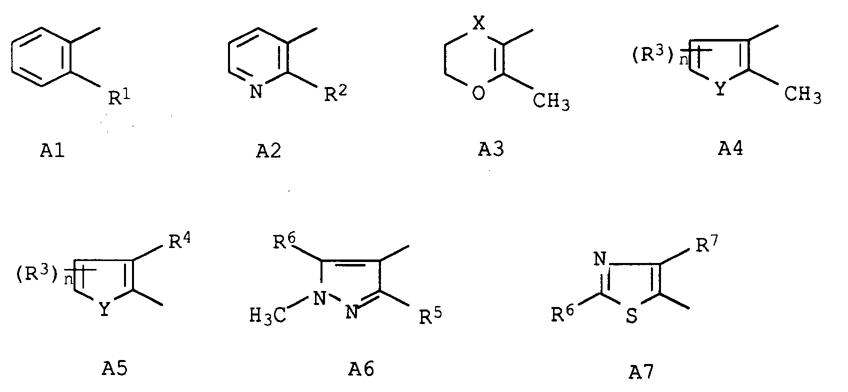 in denen die Substituenten die folgende Bedeutung haben:
- X: -CH₂-, -S-, -SO- oder SO₂-;
- Y: -O- oder -S-;
- R¹, R², R⁴, R⁵ und R⁷: Halogen, C₁-C₄-Alkyl oder C₁-C₄-Halogenalkyl;
- R³ und R⁶: Wasserstoff, Halogen oder C₁-C₄-Alkyl;
- n: 1 oder 2, wobei die Reste R³ verschieden sein können, wenn der Wert von n 2 beträgt.

Außerdem betrifft die Erfindung Verfahren zur Herstellung dieser Verbindungen, sie enthaltende Mittel und Verfahren zu deren Verwendung zur Bekämpfung von Schadpilzen.

Aus der Literatur N-Cyclohexyl-carbonsäuresäureamide mit fungiziden Eigenschaften bekannt (z.B. N-(2-Methylcyclohexyl)-2-chlornicotinsäureamid aus DE-A 24 17 216; N-Cyclohexyl-2-methylbenzoesäureamid, N-Cyclohexyl-3-methylthiophen-2-carbonsäureamid, N-Cyclohexyl-2,5-dimethylfuran-3-carbonsäureamid, N-Cyclohexyl-2-methyl-5,6-dihydro-1,4-oxathiin-3-carbonsäureamid aus Pestic. Biochem. Physiol., 34, 255 (1989)).

Aufgabe der vorliegenden Erfindung waren neue fungizid wirksame Verbindungen mit verbessertem Wirkungsspektrum, insbesondere gegen Botrytis.

Demgemäß wurden die eingangs definierten Verbindungen I gefunden. Außerdem wurden Verfahren zur Herstellung dieser Verbindungen, sie enthaltende Mittel und Verfahren zu deren Verwendung zur Bekämpfung von Schadpilzen gefunden.

Man erhält die Verbindungen I im allgemeinen dadurch, daß man ein Carbonsäurehalogenid der Formel II in an sich bekannter Weise (z.B. J. March, Advanced Organic Chemistry, 2nd Ed., 382 f, McGraw-Hill, 1977) in Gegenwart einer Base mit einem Cyclohexylamin der Formel III umsetzt. 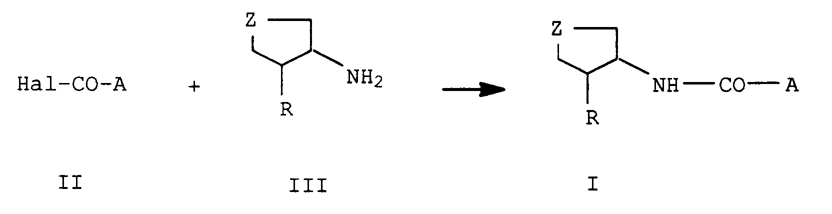
Der Rest Hal in der Formel II steht für ein Halogenatom wie Chlor, Brom und Jod, insbesondere Chlor oder Brom.

Diese Umsetzung erfolgt üblicherweise bei Temperaturen von -20 °C bis 100 °C, vorzugsweise 0 °C bis 50 °C.

Geeignete Lösungsmittel sind:
Aliphatische Kohlenwasserstoffe wie Pentan, Hexan, Cyclohexan und Petrolether, aromatische Kohlenwasserstoffe wie Toluol, o-, m-und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Toluol, Xylol und Methylenchlorid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, und metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Triethylamin und Pyridin.

Die Basen werden im allgemeinen in äquimolarem Mengen bezogen auf die Verbindung II eingesetzt. Sie können aber auch in einem Über schuß von 5 mol-% bis 30 mol-%, vorzugsweise 5 mol-% bis 10 mol-%, oder - im Falle der Verwendung von tertiären Aminen - gegebenenfalls als Lösungsmittel verwendet werden.

Die Edukte werden im allgemeinen in äquimolaren Mengen miteinander umgesetzt. Es kann für die Ausbeute vorteilhaft sein, II in einem Überschuß von 1 mol-% bis 20 mol-%, vorzugsweise 1 mol-% bis 10 mol-%, bezogen auf III einzusetzen.

Die für die Herstellung der Verbindungen I benötigten Ausgangsstoffe der Formel III sind in der Literatur bekannt (Tetrahedron Lett., Vol. 32, 1695 (1991); Houben Weyl, Methoden der org. Chemie, Bd. 11/1, S. 382 f. & 611 f.; J. Chem. Soc. C. 10, 1805 (1971); J. Org. Chem. 53, 4852 (1988); Tetrahedron 23, 2421 (1967); Tetrahedron 47, 3075 (1991)) oder können gemäß der zitierten Literatur hergestellt werden. Die bei der Reaktion z.T. anfallenden cis/trans Gemische der Verbindungen III können im allgemeinen destillativ getrennt werden.

Im Hinblick auf ihre Verwendung in fungiziden Mitteln kommen Verbindungen der Formel I in Betracht, in der die Substituenten die folgende Bedeutung haben:
- R: C₂-C₁₂-Alkyl wie Ethyl und geradkettiges oder verzweigtes Propyl, Butyl, Pentyl, Hexyl, Heptyl, Octyl, Nonyl, Decyl, Undecyl und Dodecyl, besonders geradkettiges oder verzweigtes C₃-C₁₀-Alkyl wie Propyl, 1-Methylethyl, Butyl, 1-Methylpropyl, 2-Methylpropyl, 1,1-Dimethylethyl, n-Pentyl, 1-Methylbutyl, 2-Methylbutyl, 3-methylbutyl, 1,2-Dimethylpropyl, 1,1-Dimethylpropyl, 2,2-Dimethylpropyl, 1-Ethylpropyl, n-Hexyl, 1-Methpentyl, 2-Methylpentyl, 3-Methylpentyl, 4-Methylpentyl, 1,2-Dimethylbutyl, 1,3-Dimethylbutuyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-3-methylpropyl, n-Heptyl, 1-Methylhexyl, 1-Ethylpentyl, 2-Ethylpentyl, 1-Propylbutyl, Octyl, 1-Methylheptyl, 2-Methylheptyl, 1-Ethylhexyl, 2-Ethylhexyl, 1-Propylpentyl, 2-Propylpentyl, Nonyl, 1-Methyloctyl, 2-Methyloctyl, 1-Ethylheptyl, 2-Ethylheptyl, 1-Propylhexyl, 2-Propylhexyl, Decyl, 1-Methylnonyl, 2-Methylnonyl, 1-Ethyloctyl, 2-Ethyloctyl, 1-Propylheptyl und 2-Propylheptyl, insbesondere Propyl, 1-Methylethyl, Butyl, 1-Methylbutyl, 2-Methylbutyl, 1,1-Dimethylethyl, Pentyl, 1-Methylbutyl, Hexyl, Heptyl und 1-Methylheptyl, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2, 2-Dichlor-2-fluorethyl, 2,2,2-Trichlorethyl und Pentafluorethyl;
C₂-C₁₂-Alkoxy wie Ethoxy und geradkettiges oder verzweigtes Propyloxy, Butyloxy, Pentyloxy, Hexyloxy, Heptyloxy, Octyloxy, Nonyloxy, Decyloxy, Undecyloxy und Dodecyloxy, besonders geradkettiges oder verzweigtes C₂-C₁₀-Alkoxy wie Ethoxy, Propoxy, 1-Methylethoxy, Butoxy, 1-Methylpropoxy, 2-Methylpropoxy, 1,1-Dimethylethoxy, n-Pentyloxy, 1-Methylbutoxy, 2-Methylbutoxy, 3-Methylbutoxy, 1,2-Dimethylpropoxy, 1-Ethylpropoxy, n-Hexyloxy, 1-Methylpentyloxy, 2-Methylpentyoxy, 3-Methylpentyloxy, 4-Methylpentyloxy, 1,2-Dimethylbutoxy, 1,3-Dimethylbutoxy, 2,3-Dimethylbutoxy, 1,2-Dimethylbutoxy, 2,2-Dimethylbutoxy, 3,3-Dimethylbutoxy, 1,1,2-Trimethylpropoxy, 1,2,2-Trimethylpropoxy, 1-Ethylbutoxy, 2-Ethylbutoxy, 1-Ethyl-2-methylpropoxy, n-Heptyloxy, 1-Methylhexyloxy, 2-Methylhexyloxy, 3-Methylhexyloxy, 4-Methylhexyloxy, 5-Methylhexyloxy, 1-Ethylpentyloxy, 2-Ethylpentyloxy, 1-Propylbutoxy, Octyloxy, 1-Methylheptyloxy, 2-Methylheptyloxy, 1-Ethylhexyloxy, 2-Ethylhexyloxy, 1-Propylpentyloxy, 2-Propylpentyloxy, Nonyloxy, 1-Methyloctyloxy, 2-Methyloctyloxy, 1-Ethylheptyloxy, 2-Ethylheptyloxy, 1-Propylhexyloxy, 2-Propylhexyloxy, Decyloxy, 1-Methylnonyloxy, 2-Methylnonyloxy, 1-Ethyloctyloxy, 2-Ethyloctyloxy, 1-Propylheptyloxy und 2-Propylheptyloxy, insbesondere Ethoxy, Propyloxy, 1-Methylethoxy, Butyloxy, 1-Methylpropyloxy, 2-Methylpropyloxy, 1,1-Dimethylethoxy, Pentyloxy, Hexyloxy und 2-Ethylhexyloxy, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
C₃-C₁₂-Alkenyl wie geradkettiges oder verzweigtes Propenyl, Butenyl, Pentenyl, Hexenyl, Heptenyl, Octenyl, Nonenyl, Decenyl, Undecenyl und Dodecenyl, besonders gradkettiges oder verzweigtes C₃-C₁₀-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 3-Hexenyl, 5-Hexenyl, 1-Methyl-2-pentenyl, 2-Methyl-2-pentenyl, 1-Methyl-3-pentenyl, 2-Methyl-3-pentenyl, 1-Methyl-2-hexenyl, 2-Methyl-2-hexeny, 1-Methyl-3-hexenyl, 2-Methyl-3-hexenyl, 1-Ethyl-2-pentenyl, 2-Ethyl-2-pentenyl, 1-Ethyl-3-pentenyl, 2-Ethyl-3-pentenyl, 1-Methyl-2-heptenyl, 2-Methyl-2-heptenyl, 1-Methyl-3-heptenyl, 2-Methyl-3-heptenyl, 1-Ethyl-2-hexenyl, 2-Ethyl-2-hexenyl, 1-Ethyl-3-hexenyl, 2-Ethyl-3-hexenyl, 1-Methyl-2-octenyl, 2-Methyl-2-octenyl, 1-Methyl-3-octenyl, 2-Methyl-3-octenyl, 1-Ethyl-2-heptenyl, 2-Ethyl-2-heptenyl, 1-Ethyl-3-heptenyl, 2-Ethyl-3-heptenyl, 1-Ethyl-2-octenyl, 2-Ethyl-2-octenyl, 1-Ethyl-3-octenyl und 2-Ethyl-3-octenyl, insbesondere 1-Propenyl, 2-Propenyl, 1-Methylethenyl, 1-Methyl-2-propenyl, 2-Methyl-2-propenyl, 1-Ethyl-2-propenyl, 1-Methyl-2-butenyl, 1-Ethyl-2-butenyl, 1-(1-Methylethyl)-2-butenyl, 1-Butyl-2-butenyl, 1-Methyl-2-pentenyl und 1,4-Dimethyl-2-pentenyl, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, insbesondere 3-Chlor-2-propenyl, 2,3-Dichlor-2-propenyl und 2,3,3-Trichlor-2-propenyl;
C₃-C₁₂-Alkenyloxy wie geradkettiges oder verzweigtes Propenyloxy, Butenyloxy, Pentenyloxy, Hexenyloxy, Heptenyloxy, Octenyloxy, Nonenyloxy, Decenyloxy, Undecenyloxy und Dodecenyloxy, besonders gradkettiges oder verzweigtes C₃-C₁₀-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenlyoxy, 1-Ethyl-1-methyl-2-propenyloxy, 1-Ethyl-2-methyl-2-propenyloxy, 1-Methyl-2-pentenyloxy, 2-Methyl-2-pentenyloxy, 1-Methyl-3-pentenyloxy, 2-Methyl-3-pentenyloxy, 1-Methyl-2-hexenyloxy, 2-Methyl-2-hexenyloxy, 1-Methyl-3-hexenyloxy, 2-Methyl-3-hexenyloxy, 1-Ethyl-2-pentenyloxy, 2-Ethyl-2-pentenyloxy, 1-Ethyl-3-pentenyloxy, 2-Ethyl-3-pentenyloxy, 1-Methyl-2-heptenyloxy, 2-Methyl-2-heptenyloxy, 1-Methyl-3-heptenyloxy, 2-Methyl-3-heptenyloxy, 1-Ethyl-2-hexenyloxy, 2-Ethyl-2-hexenyloxy, 1-Ethyl-3-hexenyloxy, 2-Ethyl-3-hexenyloxy, 1-Methyl-2-octenyloxy, 2-Methyl-2-octenyloxy, 1-Methyl-3-octenyloxy, 2-Methyl-3-octenyloxy, 1-Ethyl-2-heptenyloxy, 2-Ethyl-2-heptenyloxy, 1-Ethyl-3-heptenyloxy, 2-Ethyl-3-heptenyloxy, 1-Ethyl-2-octenyloxy, 2-Ethyl-2-octenyloxy, 1-Ethyl-3-octenyloxy und 2-Ethyl-3-octenyloxy, insbesondere 2-Propenyloxy, 1-Methyl-2-propenyloxy, 2-Methyl-2-propenyloxy, 2-Pentenyloxy, 3-Pentenyloxy, 1-Methyl-2-butenyloxy und 1-Methyl-2-pentenyloxy, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, insbesondere 3-Chlor-2-propenyloxy, 2,3-Dichlor-2-propenyloxy und 2,3,3-Trichlor-2-propenyloxy;
C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,2-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 2-Propinyl, 2-Butinyl und 3-Butinyl, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise 3-Chlor-2-propinyl, 3-Chlor-2-butinyl und 4-Chlor-3-butinyl;
C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Alkinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-3-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, vorzugsweise 2-Propinyloxy, 2-Butinyloxy, 1-Methyl-2-propinyloxy und 1-Methyl-2-butinyloxy, 2-Propinyloxy, 2-Butinyloxy, 3-Butinyloxy und 1-Methyl-2-propinyloxy, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise 3-Chlor-2-propinyloxy, 3-Chlor-2-butinyloxy und 4-Chlor-3-butinyloxy;
C₃-C₇-Cycloalkyl wie Cyclopropyl, Cyclobutyl, Cyclopentyl, Cyclohexyl und Cycloheptyl, wobei diese Ringe ein bis drei C₁-C₄-Alkylgruppen wie Methyl, Ethyl, Propyl, 1-Methylethyl, Butyl, 1-Methylpropyl, 2-Methylpropyl und 1,1-Dimethylethyl tragen können;
C₄-C₇-Cycloalkenyl wie Cyclobutenyl, Cyclopentenyl, Cyclohexenyl und Cycloheptenyl, wobei diese Ringe ein bis drei C₁-C₄-Alkylgruppen wie Methyl, Ethyl, Propyl, 1-Methylethyl, Butyl, 1-Methylpropyl, 2-Methylpropyl und 1,1-Dimethylethyl tragen können;
C₃-C₇-Cycloalkyloxy wie Cyclopropyloxy, Cyclobutyloxy, Cyclopentyloxy, Cyclohexyloxy und Cycloheptyloxy, wobei diese Ringe ein bis drei C₁-C₄-Alkylgruppen wie Methyl, Ethyl, Propyl, 1-Methylethyl, Butyl, 1-Methylpropyl, 2-Methylpropyl und 1,1-Dimethylethyl tragen können;
oder C₄-C₇-Cycloalkenyloxy wie 1-Cyclobutenyloxy, 2-Cyclobutenyloxy, 1-Cyclopentenyloxy, 2-Cyclopentenyloxy, 3-Cyclopentenyloxy, 1-Cyclohexenyloxy, 2-Cyclohexenyloxy, 3-Cyclohexenyloxy, 1-Cycloheptenyloxy, 2-Cycloheptenyloxy, 3-Cycloheptenyloxy und 4-Cycloheptenyloxy, wobei diese Ringe ein bis drei C₁-C₄-Alkylgruppen wie Methyl, Ethyl, Propyl, 1-Methylethyl, Butyl, 1-Methylpropyl, 2-Methylpropyl und 1,1-Dimethylethyl tragen können;
Phenyl, welches ein bis fünf Halogenatome wie Fluor, Chlor, Brom und Jod, insbesondere Fluor, Chlor und Brom, und/oder ein bis drei der folgenden Reste tragen kann:
C₁-C₄-Alkyl wie vorstehend genannt;
C₁-C₄-Halogenalkyl wie vorstehend genannt;
C₁-C₄-Alkoxy wie vorstehend genannt;
C₁-C₄-Halogenalkoxy wie vorstehend genannt;
C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio;
oder C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;
- A: steht für einen cyclischen Rest aus der Gruppe der Formeln A1 bis A7 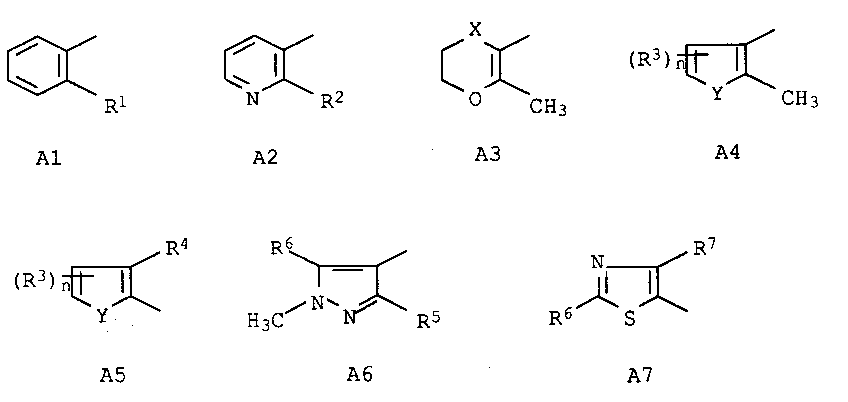 in denen die Substituenten die folgende Bedeutung haben:
- X: -CH₂-, -S-, -SO- oder -SO₂-;
- Y: -O- oder -S-;
- R¹, R², R⁴, R₅ und R⁷: unabhängig voneinander Halogen wie Fluor, Chlor und Brom, C₁-C₄-Alkyl wie vorstehend genannt, oder C₁-C₄-Halogenalkyl wie vorstehend genannt;
- R³ und R⁶: unabhängig voneinander Wasserstoff, Halogen wie Fluor, Chlor und Brom oder C₁-C₄-Alkyl wie vorstehend genannt;
- n: 1 oder 2, wobei die Reste R³ verschieden sein können, wenn der Wert von n 2 beträgt.

Im Hinblick auf die biologische Wirkung besonders bevorzugte Verbindungen der Formel I sind solche, in denen R die vorstehend gegebene Bedeutung hat und A für einen cyclischen Rest aus der Gruppe der Formeln A1 bis A7 steht, wobei X und Y die vorstehend gegebene Bedeutung und die Substituenten für die folgenden Reste stehen:
- R¹: Halogen wie Fluor, Chlor und Brom Methyl oder C₁-Halogenalkyl wie Chlormethyl, Dichlormethyl, Trichlormethyl, Fluormethyl, Difluormethyl, Trifluormethyl, Chlorfluormethyl, Dichlorfluormethyl und Chlordifluormethyl;
- R²: Halogen wie Fluor, Chlor und Brom oder C₁-Halogenalkyl wie Chlormethyl, Dichlormethyl, Trichlormethyl, Fluormethyl, Difluormethyl, Trifluormethyl, Chlorfluormethyl, Dichlorfluormethyl und Chlordifluormethyl;
- R³: Wasserstoff oder Methyl;
- n: 1 oder 2, wobei die Reste R³ verschieden sein können, wenn der Wert von n 2 beträgt;
- R⁴: Halogen wie Fluor, Chlor und Brom oder Methyl;
- R⁵: Methyl oder C₁-Halogenalkyl wie Chlormethyl, Dichlormethyl, Trichlormethyl, Fluormethyl, Difluormethyl, Trifluormethyl, Chlorfluormethyl, Dichlorfluormethyl und Chlordifluormethyl;
- R⁶: Wasserstoff, Halogen wie Fluor, Chlor und Brom oder Methyl;
- R⁷: Halogen wie Fluor, Chlor und Brom Methyl oder C₁-Halogenalkyl wie Chlormethyl, Dichlormethyl, Trichlormethyl, Fluormethyl, Difluormethyl, Trifluormethyl, Chlorfluormethyl, Dichlorfluormethyl und Chlordifluormethyl.

Insbesondere sind solche Verbindungen der Formel I bevorzugt, in denen der R die vorstehend gegebene Bedeutung hat und A für einen cyclischen Rest aus der Gruppe der Formeln A1 bis A7 steht, wobei X und Y die vorstehend gegebene Bedeutung und die Substituenten für die folgenden Gruppen stehen:
- R¹: Chlor, Brom, Jod, Methyl oder Trifluormethyl;
- R²: Chlor oder Trifluormethyl;
- R³: Wasserstoff oder Methyl;
- n: 1 oder 2, wobei die Reste R³ verschieden sein können, wenn der Wert von n 2 beträgt;
- R⁴: Chlor oder Methyl;
- R⁵: Methyl, Difluormethyl oder Trifluormethyl;
- R⁶: Wasserstoff, Chlor oder Methyl;
- R⁷: Chlor, Methyl oder Trifluormethyl.

Im Hinblick auf die biologische Wirkung sind insbesondere auch solche Verbindungen 1 bevorzugt, in denen die Gruppen R und NHCOA trans zueinander angeordnet sind.

Besonders bevorzugte Verbindungen der Formel I sind:
- Verbindungen I, in denen
   - R: für Ethyl, Propyl, iso-Propyl, Butyl, iso-Butyl, sek.-Butyl, 2-Ethylbutyl, Cyclopentyl, Cyclohexyl, Cyclopent-2-en-1-yl, Cyclohexen-1-yl, Phenyl oder Benzyl steht, wobei die Phenylreste jeweils noch eine bis drei der folgenden Gruppen tragen können: Halogen, C₁-C₄-Alkyl, C₁-C₄-Halogenalkyl, C₁-C₄-Alkoxy, C₁-C₄-Halogenalkoxy und C₁-C₄-Alkylthio,
   vorzugsweise Verbindungen I, in denen
   - R: für Ethyl, Propyl, iso-Propyl, Butyl, iso-Butyl, sek.-Butyl, Cyclohexyl, Cyclohexen-1-yl, Phenyl oder Benzyl steht, wobei die Phenylreste jeweils noch eine bis drei der folgenden Gruppen tragen können: Halogen, C₁-C₂-Alkyl, C₁-C₂-Halogenalkyl, C₁-C₂-Alkoxy, C₁-C₂-Halogenalkoxy und C₁-C₂-Alkylthio,
   insbesondere Verbindungen I, in denen
   - R: für Ethyl, Propyl, iso-Propyl, Butyl, iso-Butyl, sek.-Butyl, Cyclohexyl, Cyclohexen-1-yl, Phenyl oder Benzyl steht, wobei die Phenylreste jeweils noch eine bis drei der folgenden Gruppen tragen können: Halogen, C₁-C₂-Alkyl, C₁-C₂-Halogenalkyl, C₁-C₂-Alkoxy und C₁-C₂-Halogenalkoxy.
- Verbindungen I, in denen
   - A: für A1, A2, A3, A4, A6 oder A7 steht,
   vorzugsweise Verbindungen I, in denen
   - A: für A1, A2, A3, A4 (Y = O), A6 oder A7 steht.
- Verbindungen I, in denen
   - A: für A1 steht,
   vorzugsweise Verbindungen I, in denen
   - A: für A1 steht und
   - R¹: für Chlor, Brom, Methyl und Trifluormethyl steht und
   insbesondere Verbindungen I, in denen
   - A: für A1 steht und
   - R¹: für Brom, Methyl und Trifluormethyl steht und
   - R: für sek.-Butyl, Cyclopent-2-en-1-yl und Phenyl steht.
- Verbindungen I, in denen
   - A: für A2 steht,
   vorzugsweise Verbindungen I, in denen
   - A: für A2 und
   - R²: für Chlor steht und
   insbesondere Verbindungen I, in denen
   - A: für A2,
   - R²: für Chlor,
   - Z: für CH₂CH₂ und
   - R: für Ethyl, Propyl, iso-Propyl, Butyl, iso-Butyl, sek.-Butyl, Cyclohexyl, Cyclohexen-1-yl, Phenyl oder Benzyl steht, wobei die Phenylreste jeweils noch eine bis drei der folgenden Gruppen tragen können: Halogen, C₁-C₄-Alkyl, C₁-C₄-Halogenalkyl und C₁-C₄-Alkoxy.
- Verbindungen I, in denen
   - A: für A3 steht,
   vorzugsweise Verbindungen I, in denen
   - A: für A3
   - X: für Sauerstoff und Schwefel und
   - Z: für CH₂CH₂ steht,
   insbesondere Verbindungen I, in denen
   - A: für A3,
   - X: für Sauerstoff und Schwefel,
   - Z: für CH₂CH₂ und
   - R: für sek.-Butyl steht.
- Verbindungen I, in denen
   - A: für A4 und
   - Y: für Sauerstoff steht,
   vorzugsweise Verbindungen I, in denen
   - A: für A4 und
   - Y: für Sauerstoff und
   - R³: für Methyl steht.
   insbesondere Verbindungen I, in denen
   - A: für A4,
   - Y: für Sauerstoff,
   - R³: für Methyl,
   - Z: für CH₂CH₂ und
   - R: für sek.-Butyl und Cyclohexen-1-yl steht.
- Verbindungen I, in denen
   - A: für A6 steht,
   vorzugsweise Verbindungen I, in denen
   - A: für A6 und
   - R⁵ und R⁶: für Methyl stehen,
   insbesondere Verbindungen I, in denen
   - A: für A6,
   - R⁵ und R⁶: für Methyl,
   - Z: für CH₂CH₂ und
   - R: für Cyclohexen-1-yl steht.
- Verbindungen I, in denen
   - A: für A7 steht,
   vorzugsweise Verbindungen I, in denen
   - A: für A7 und
   - R⁶ und R⁷: unabhängig voneinander für Methyl und Trifluormethyl stehen,
   insbesondere Verbindungen I, in denen
   - A: für A7 und
   - R⁶ und R⁷: unabhängig voneinander für Methyl und Trifluormethyl,
   - Z: für CH₂CH₂ und
   - R: für Propyl, Butyl, sek.-Butyl, Cyclohexyl, Cyclohexen-1-yl, Phenyl oder Benzyl steht, wobei die Phenylreste jeweils noch eine bis drei der folgenden Gruppen tragen können: Halogen, C₁-C₄-Alkyl, C₁-C₄-Halogenalkyl und C₁-C₄-Alkoxy.

Besonders bevorzugte Verbindungen der Formel I sind in den folgenden Tabellen A bis G zusammengestellt.

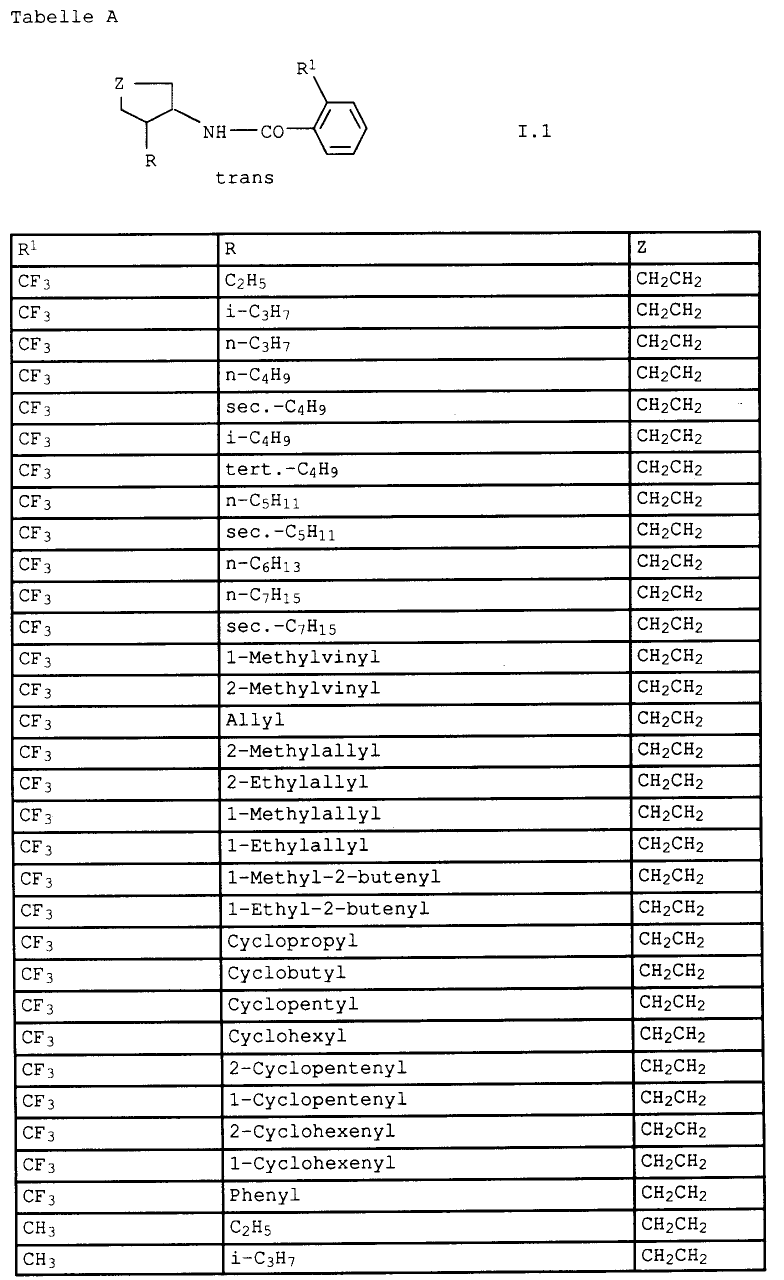

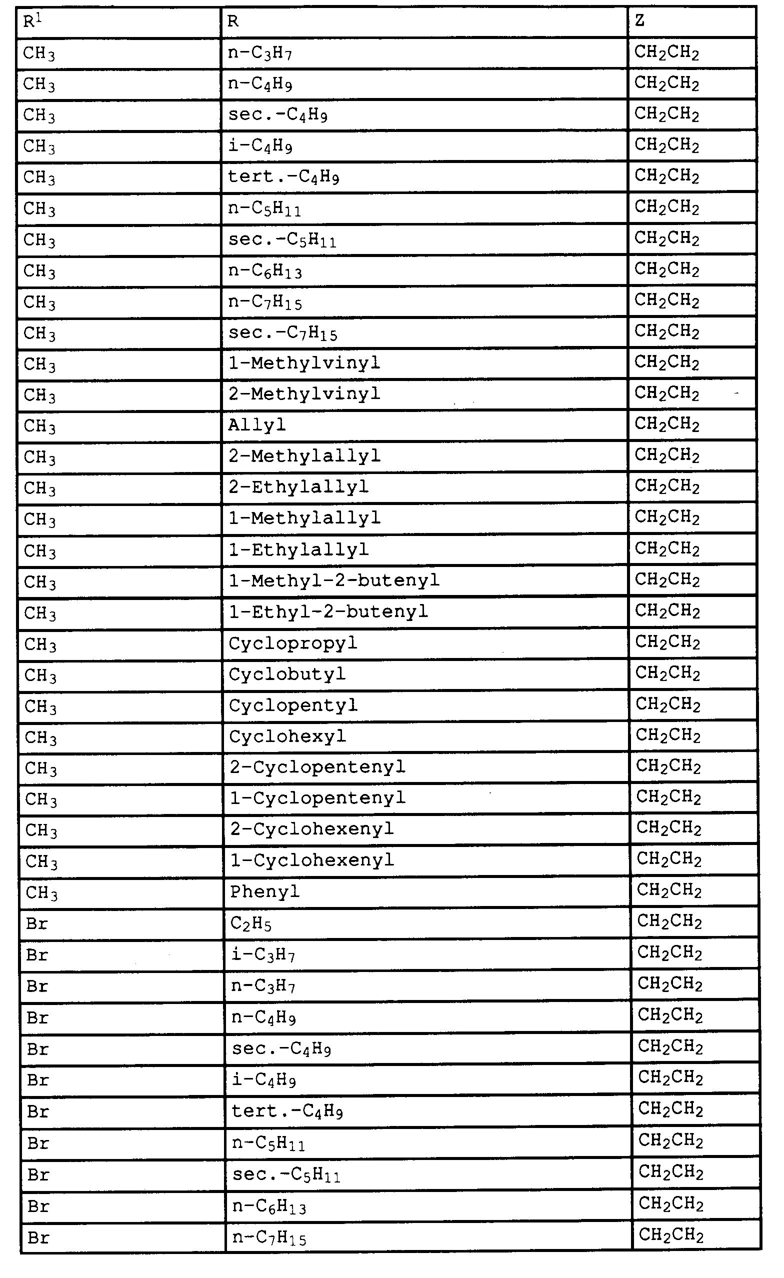

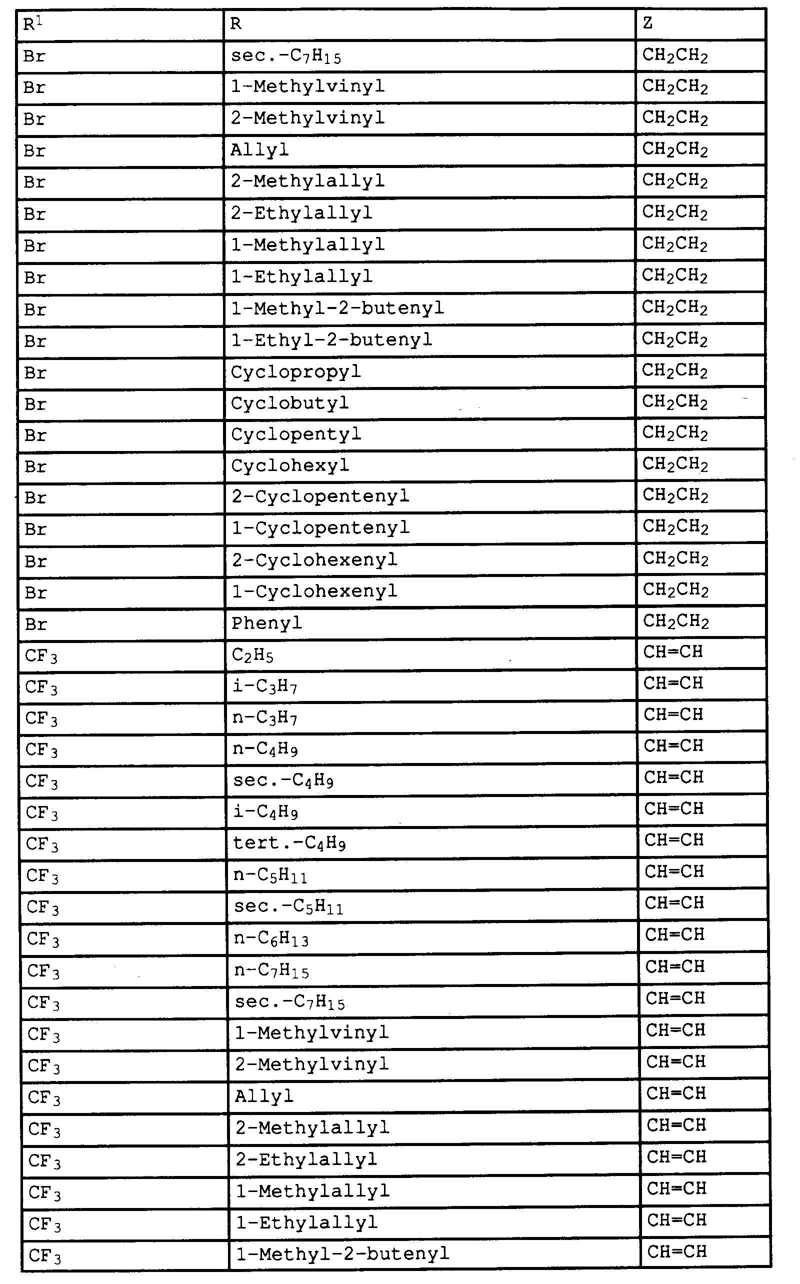

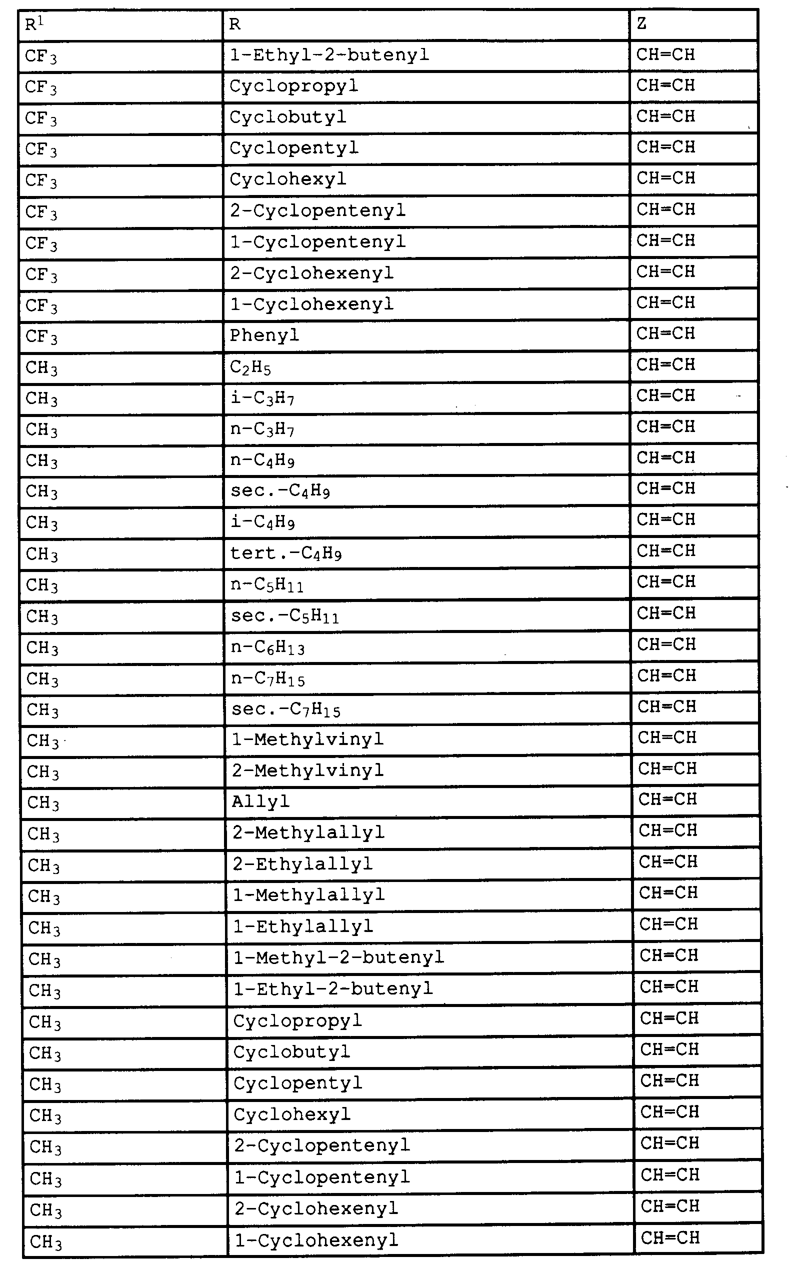

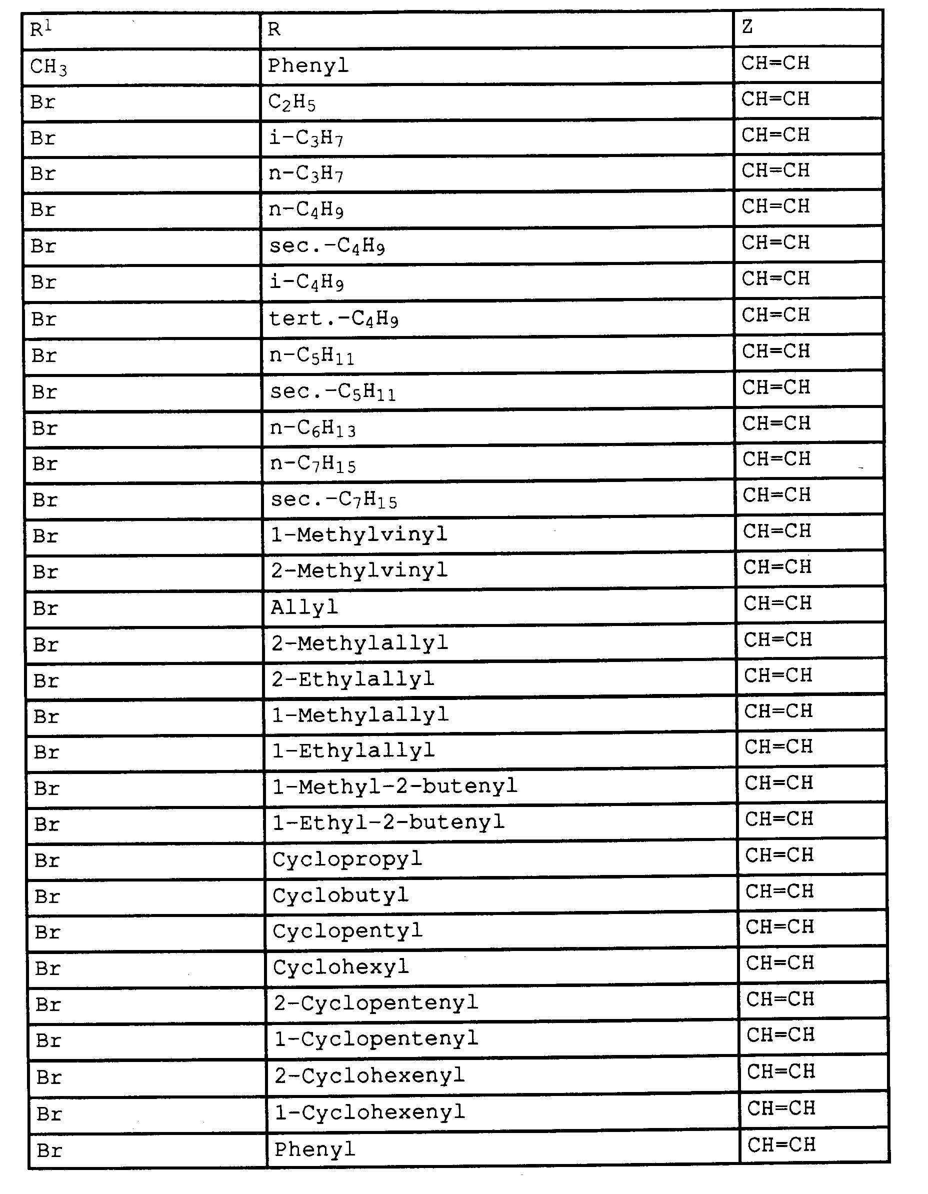

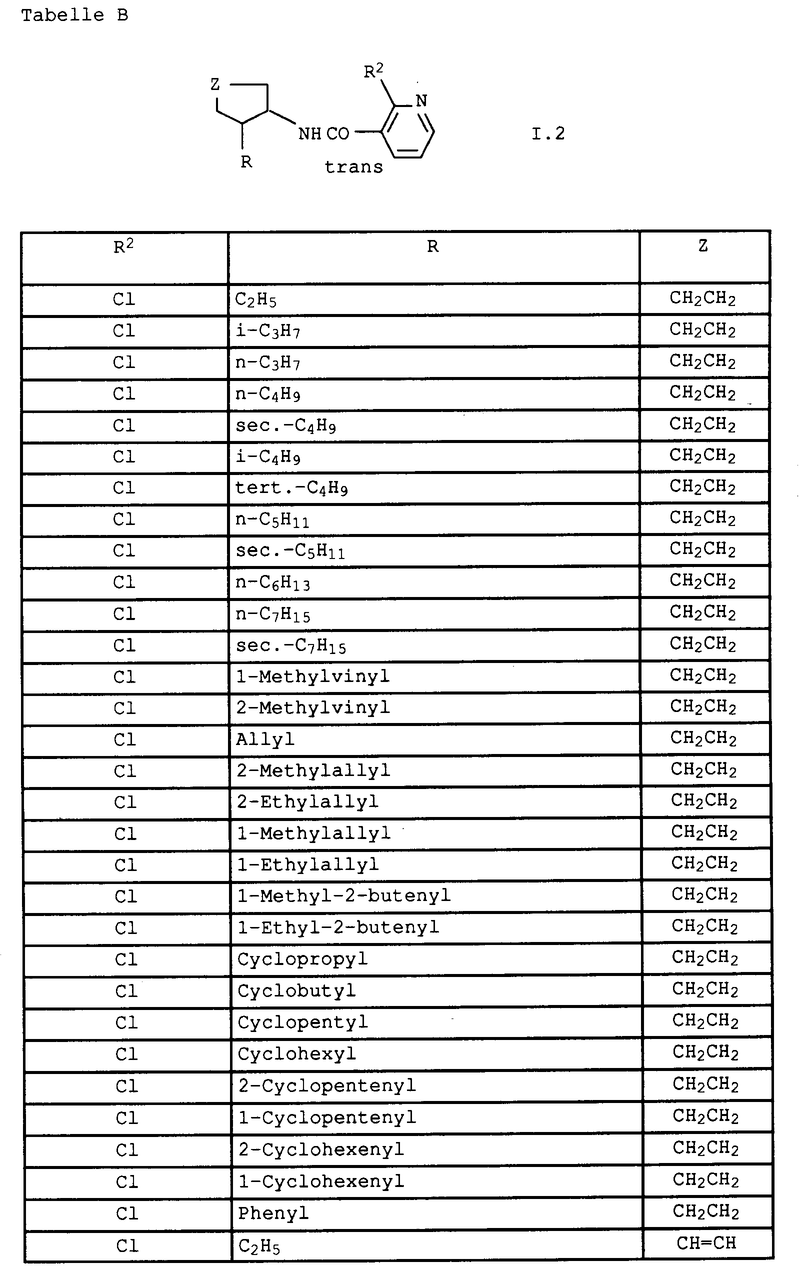

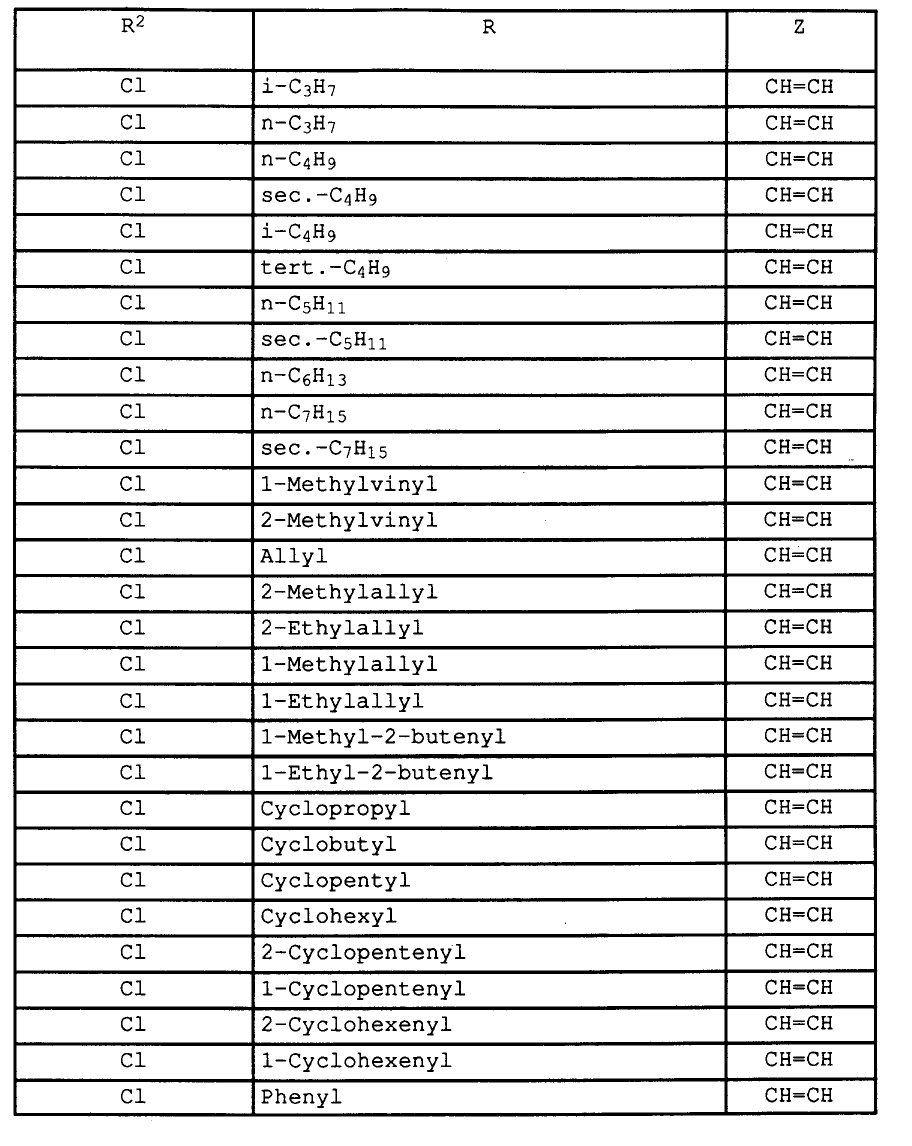

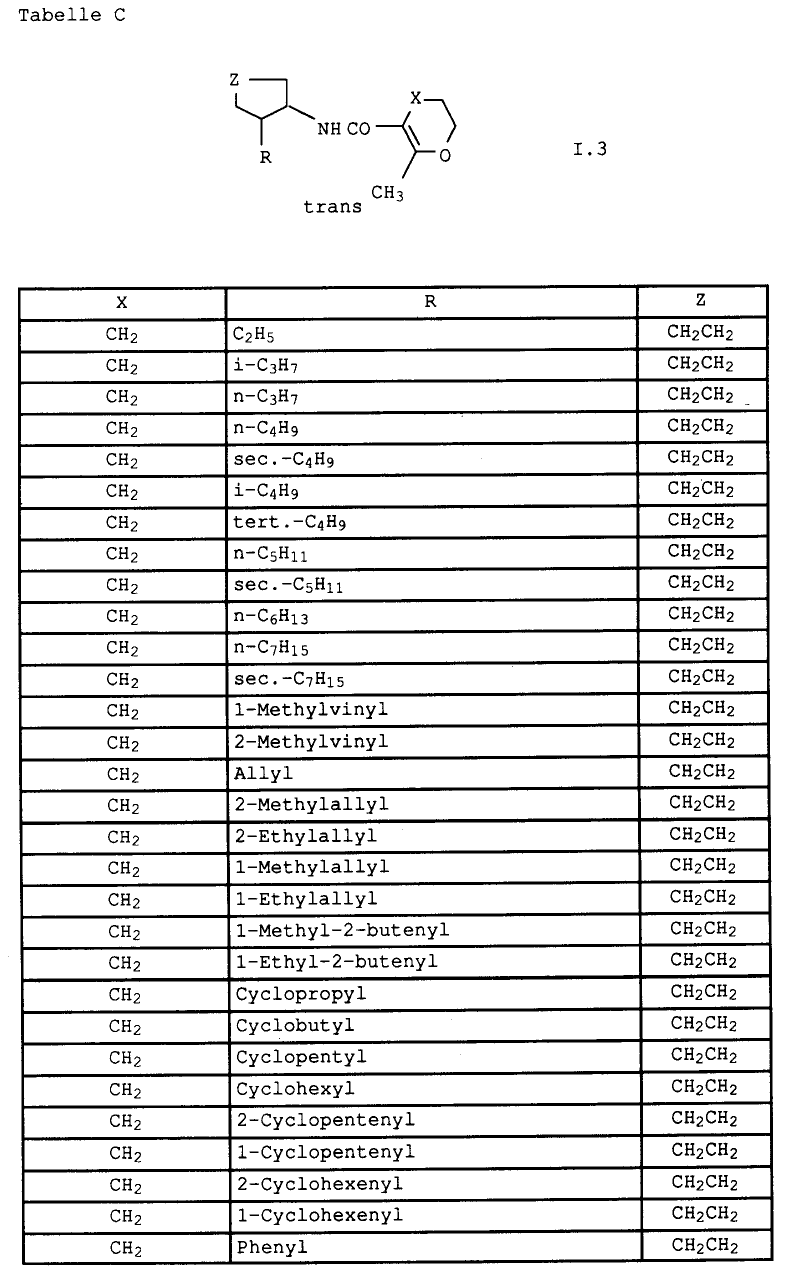

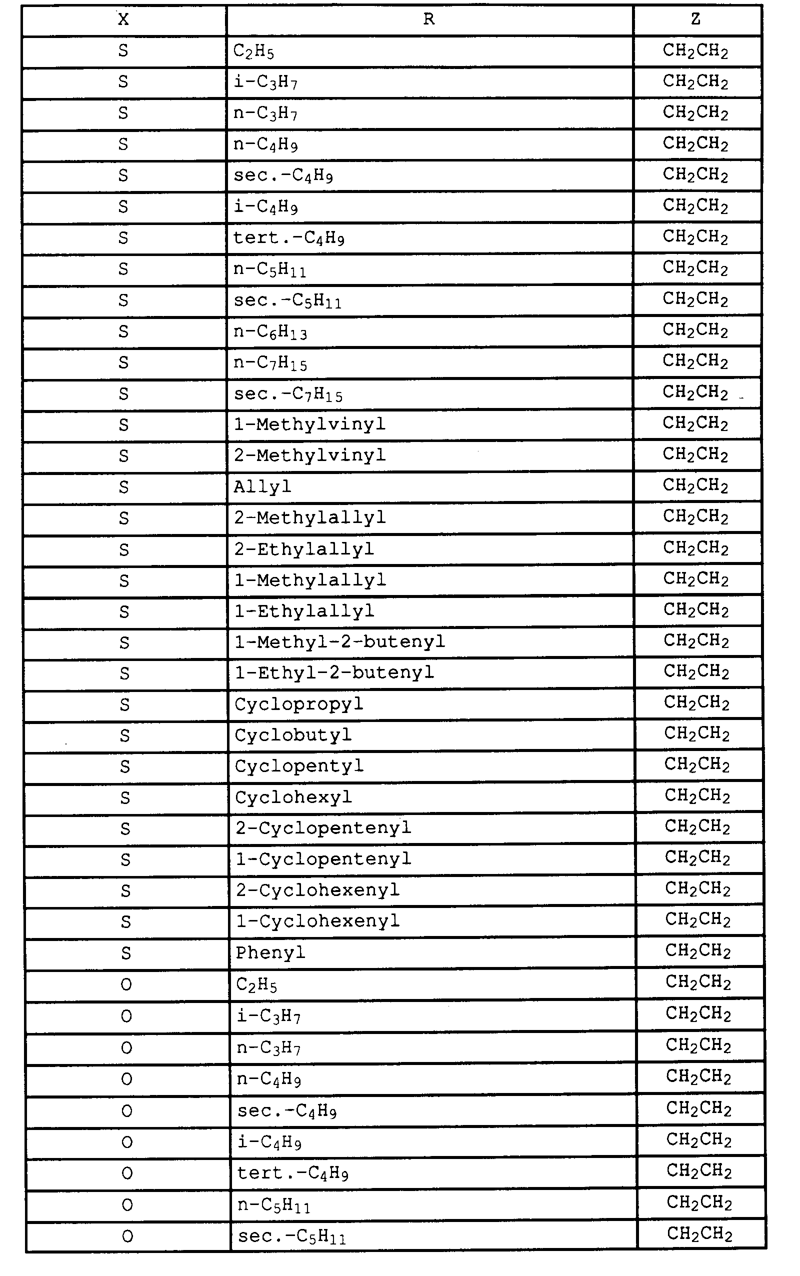

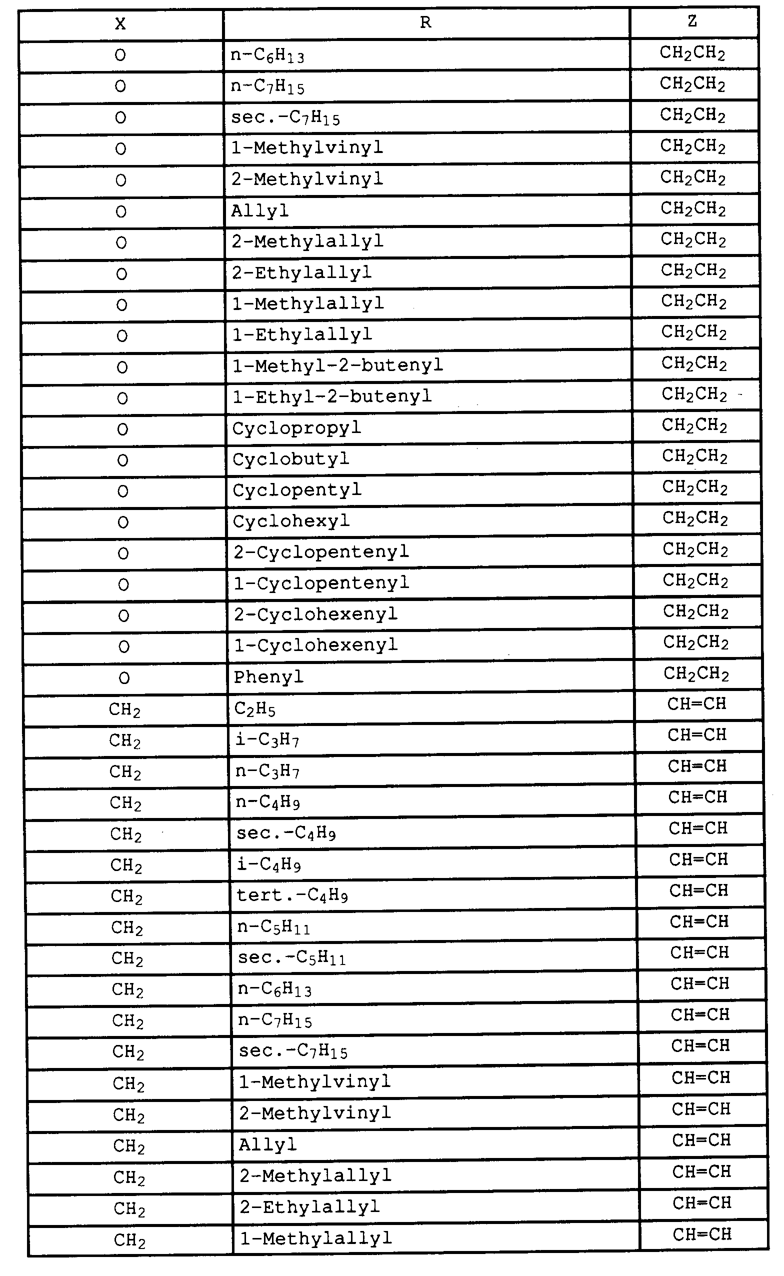

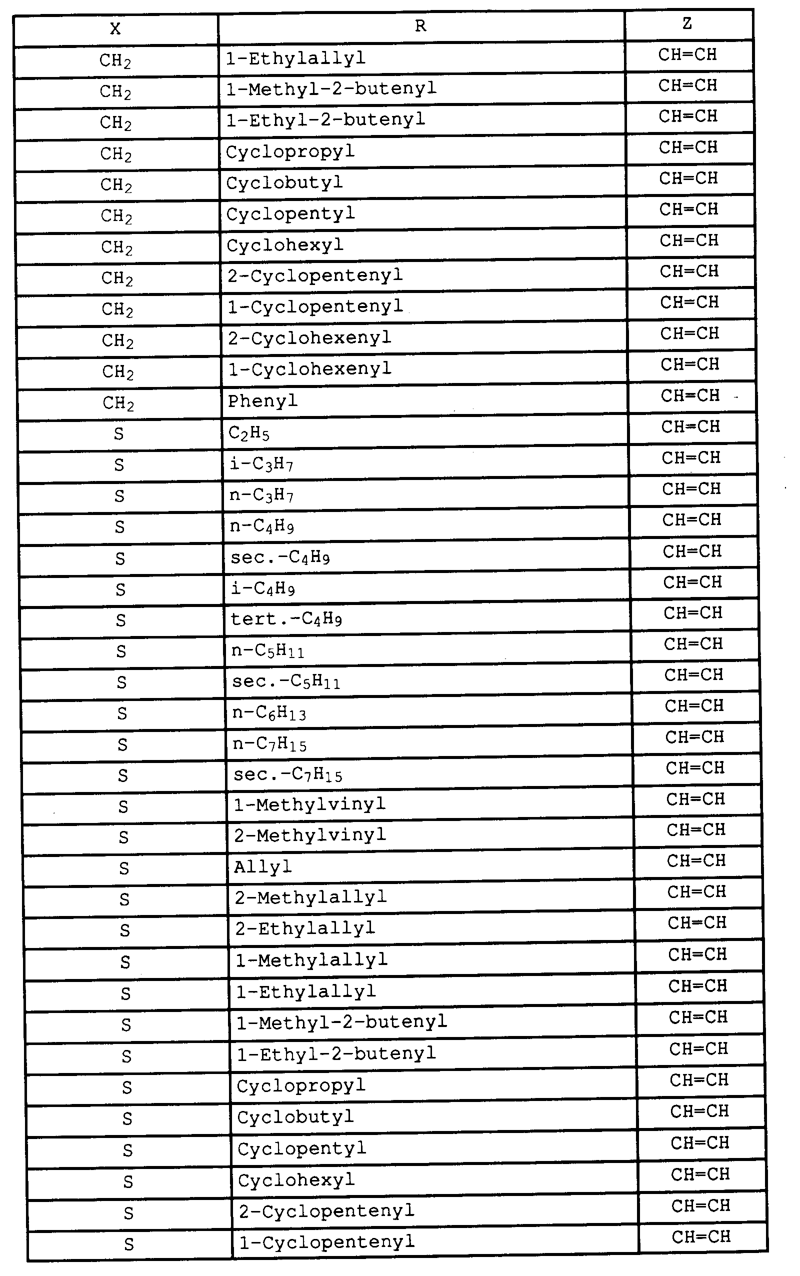

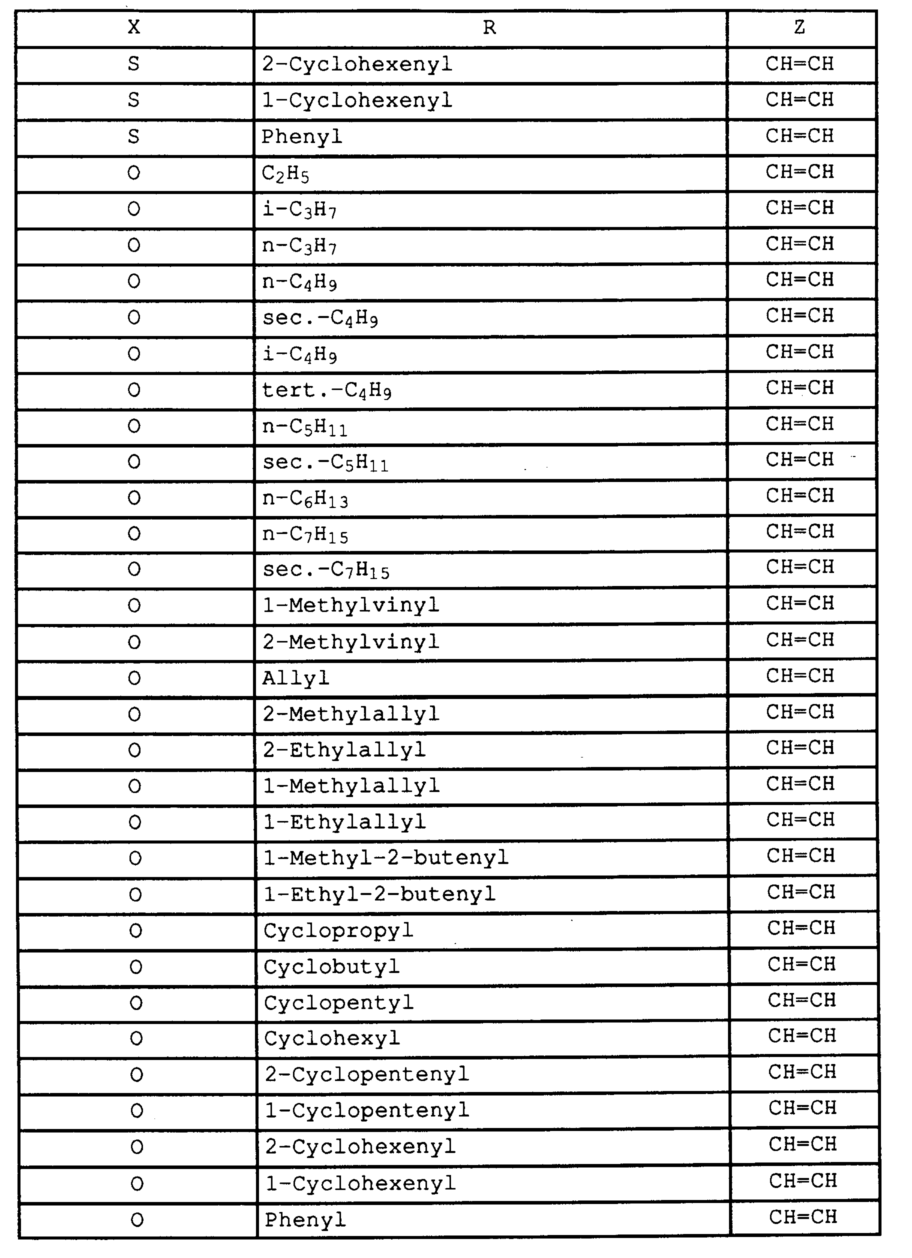

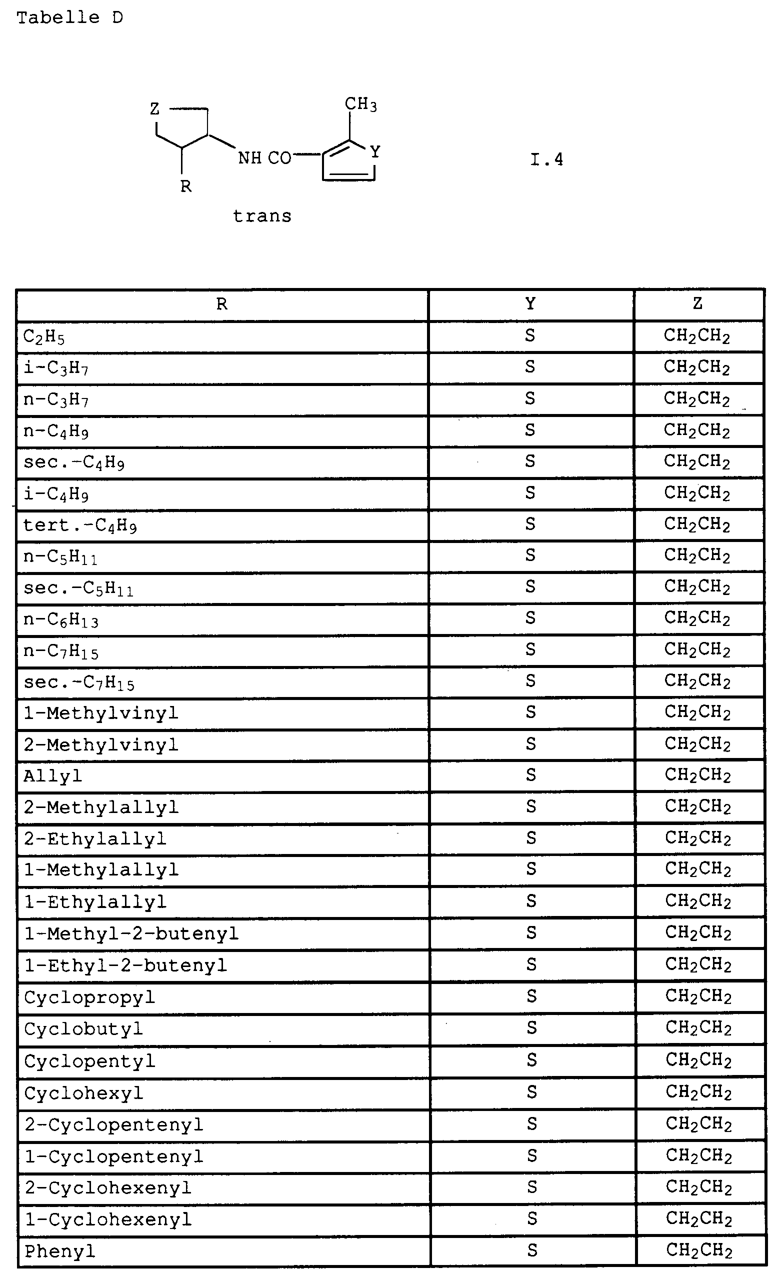

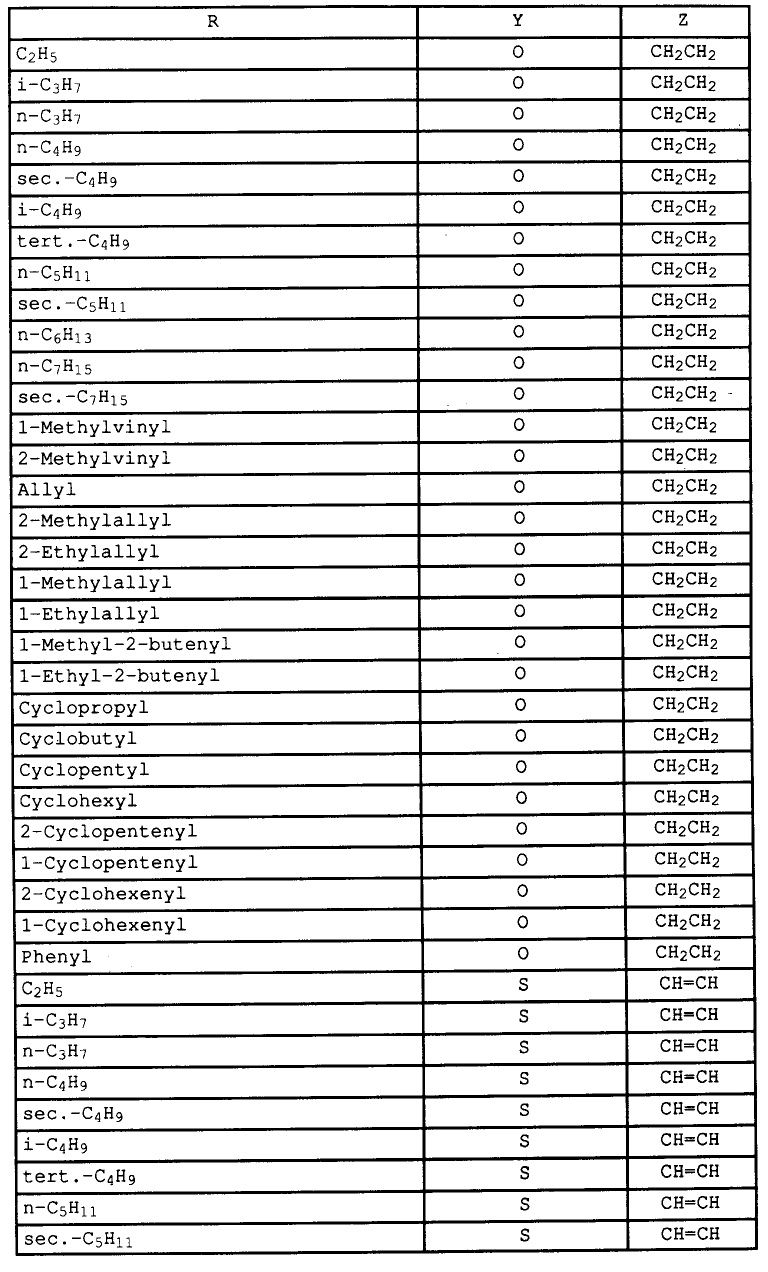

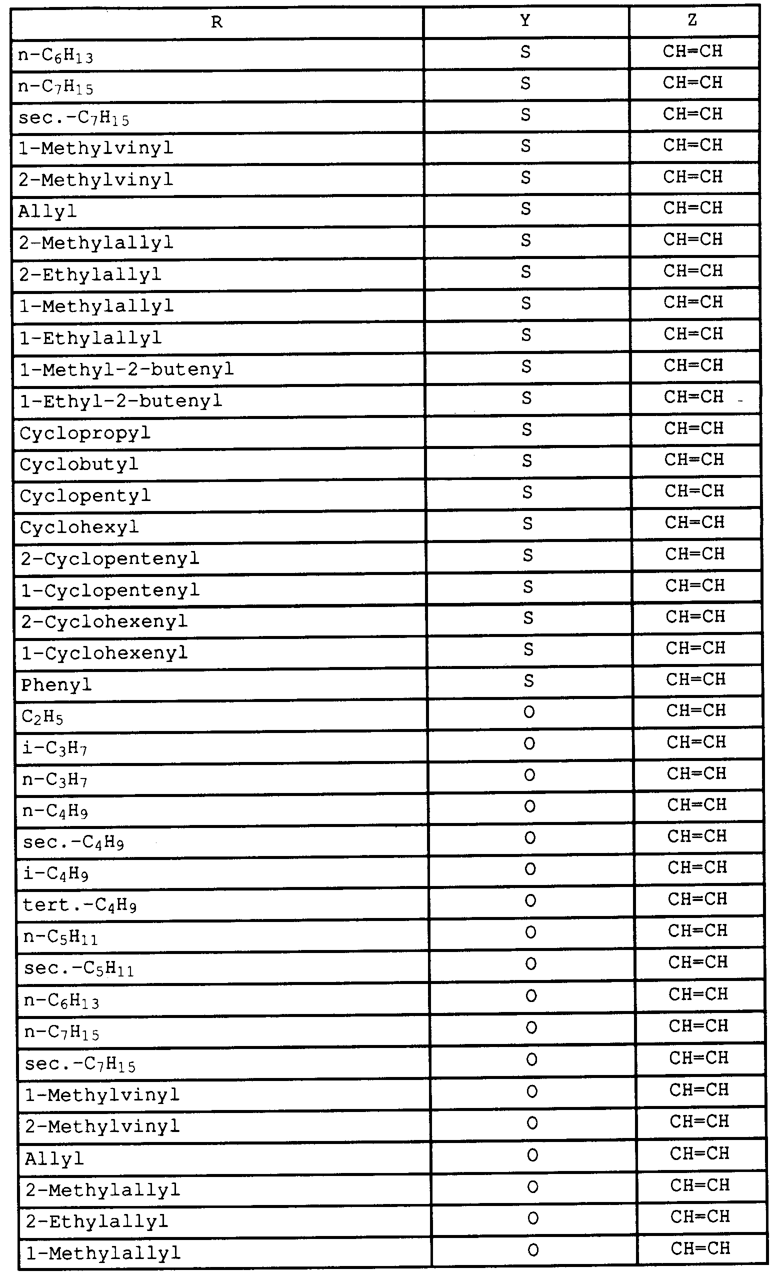

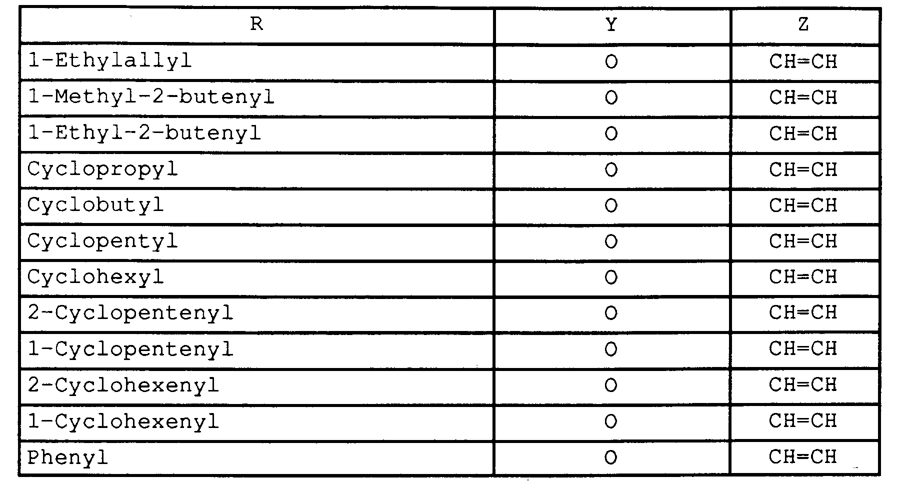

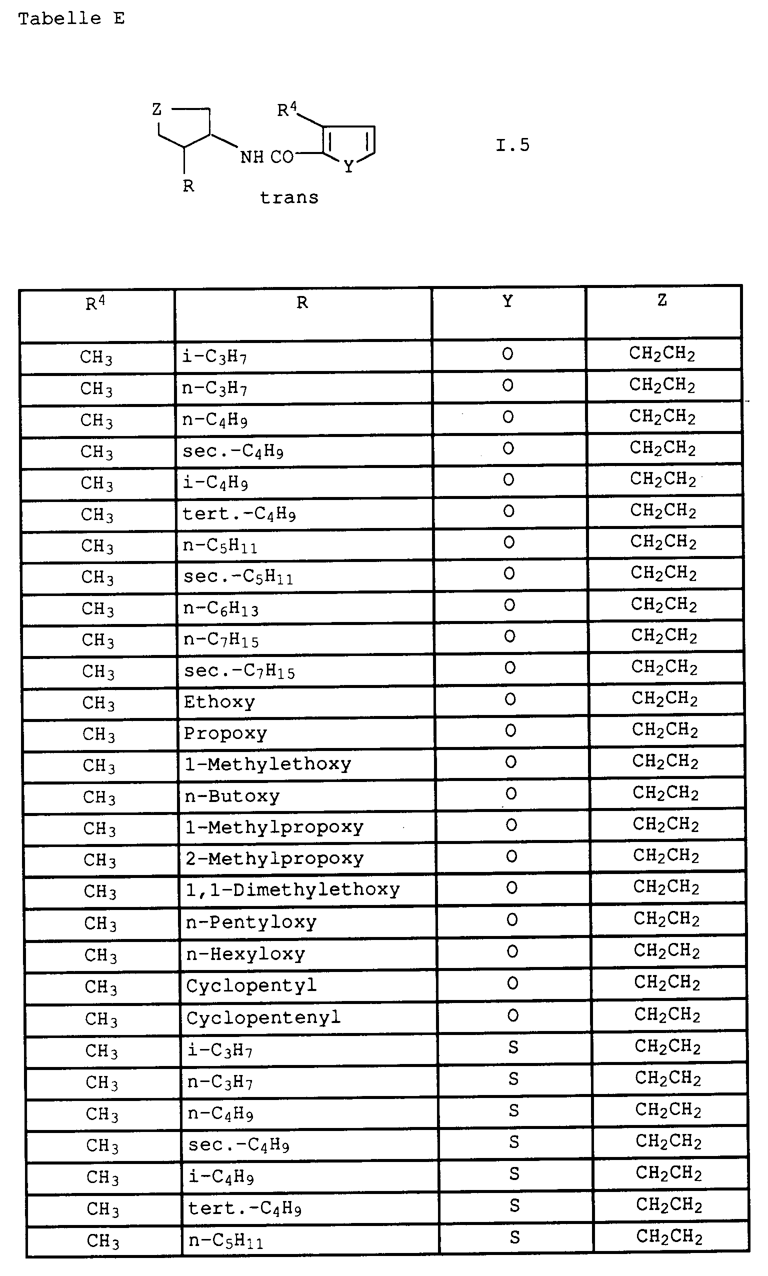

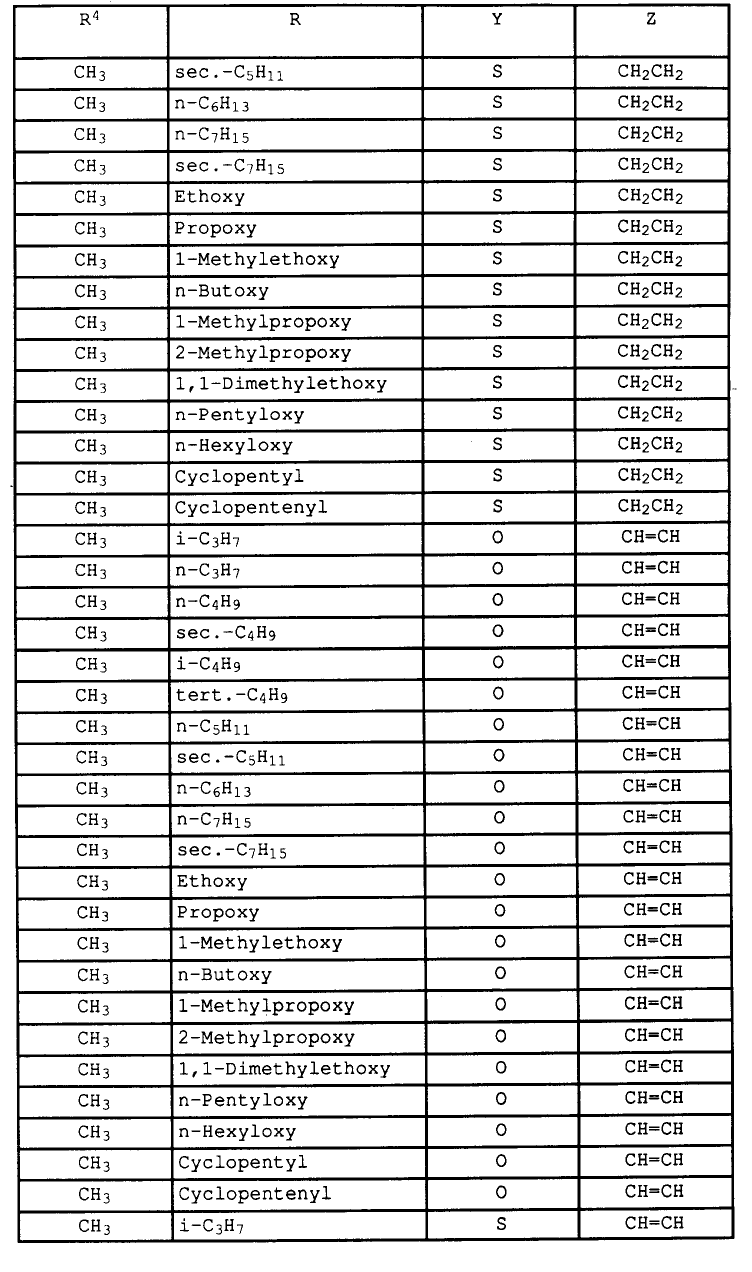

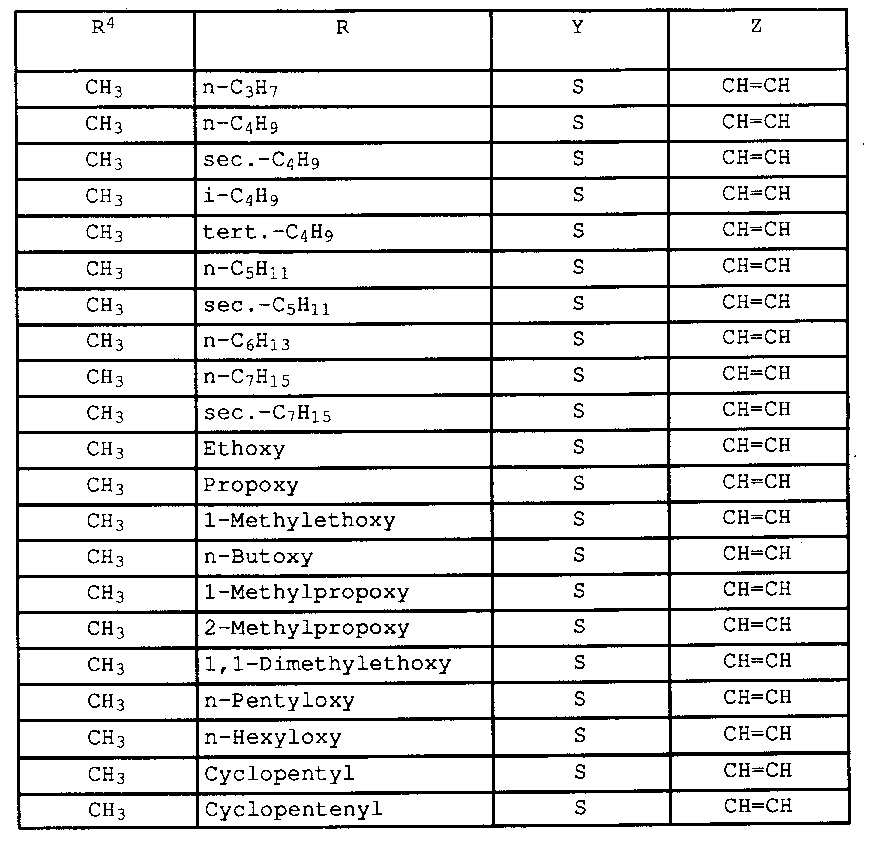

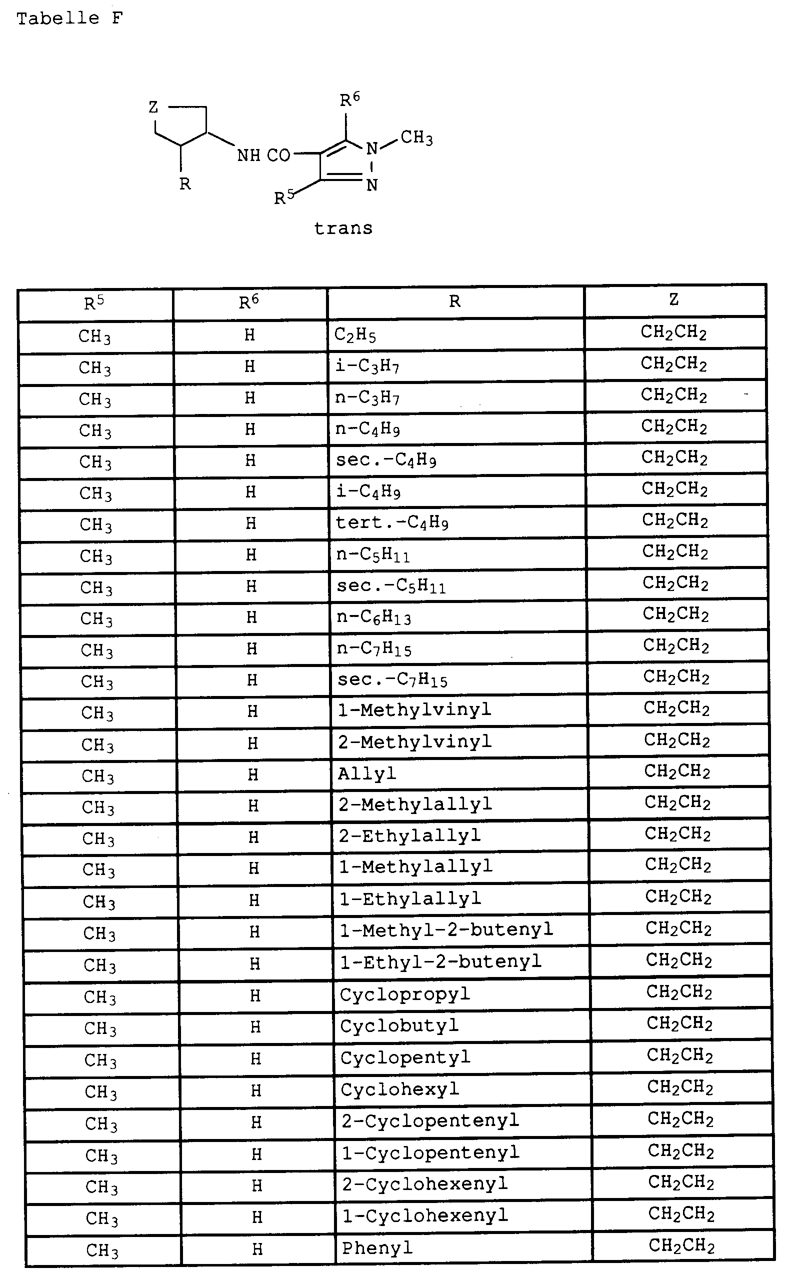

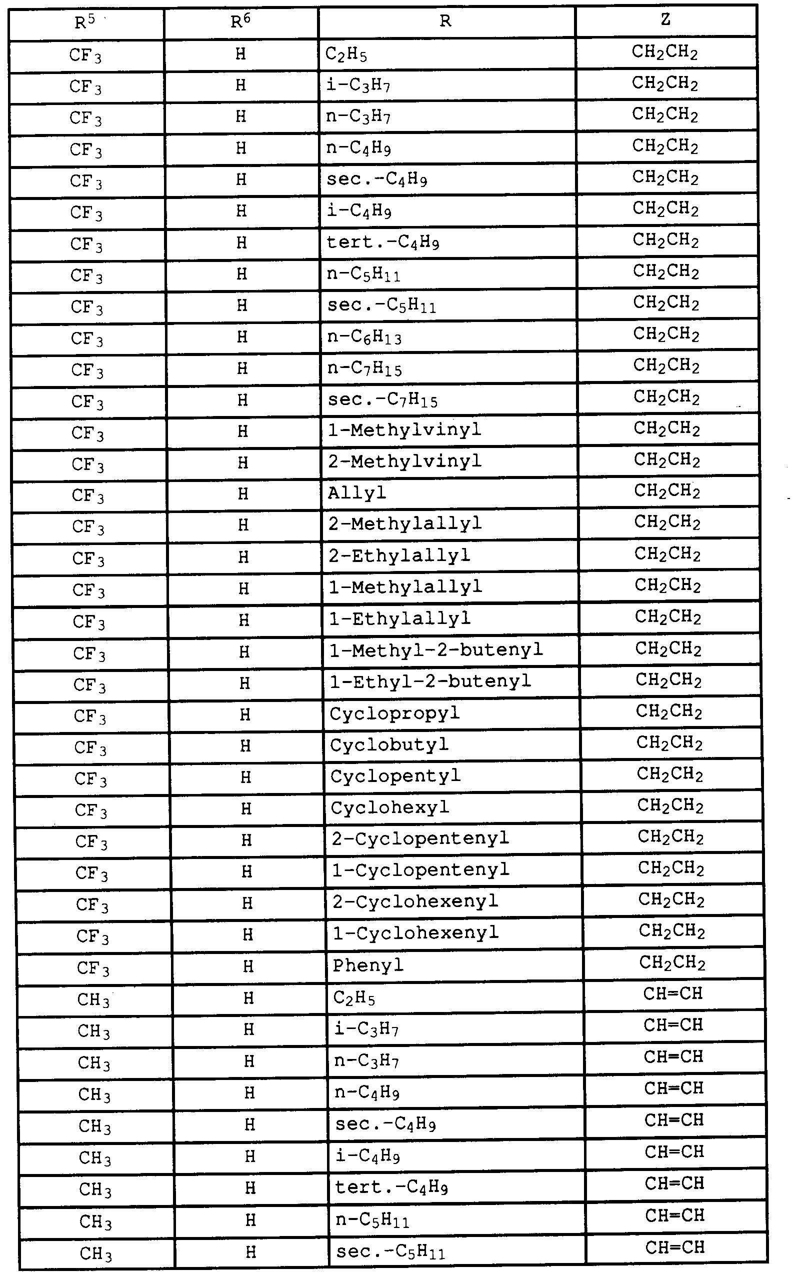

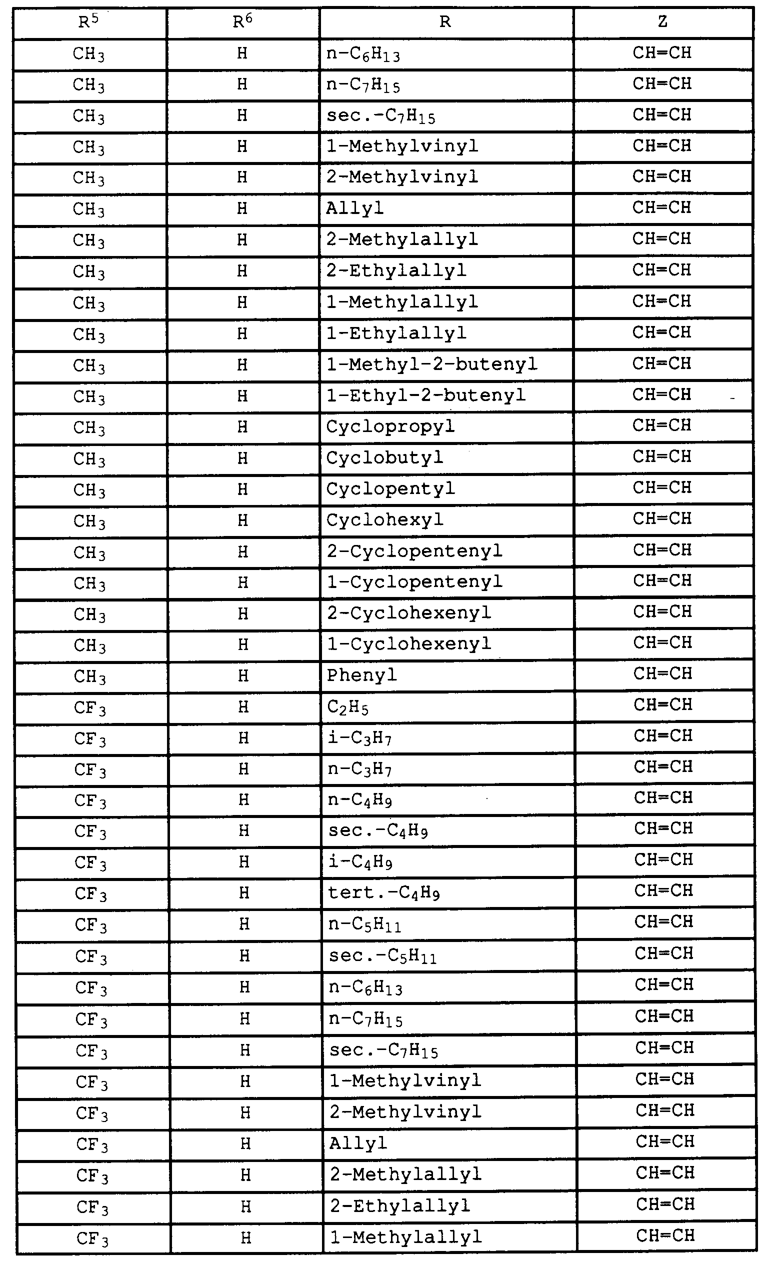

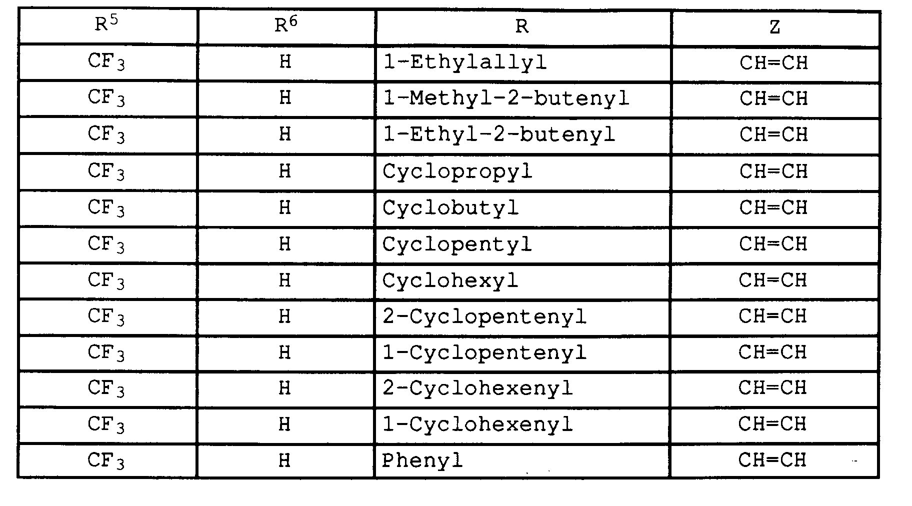

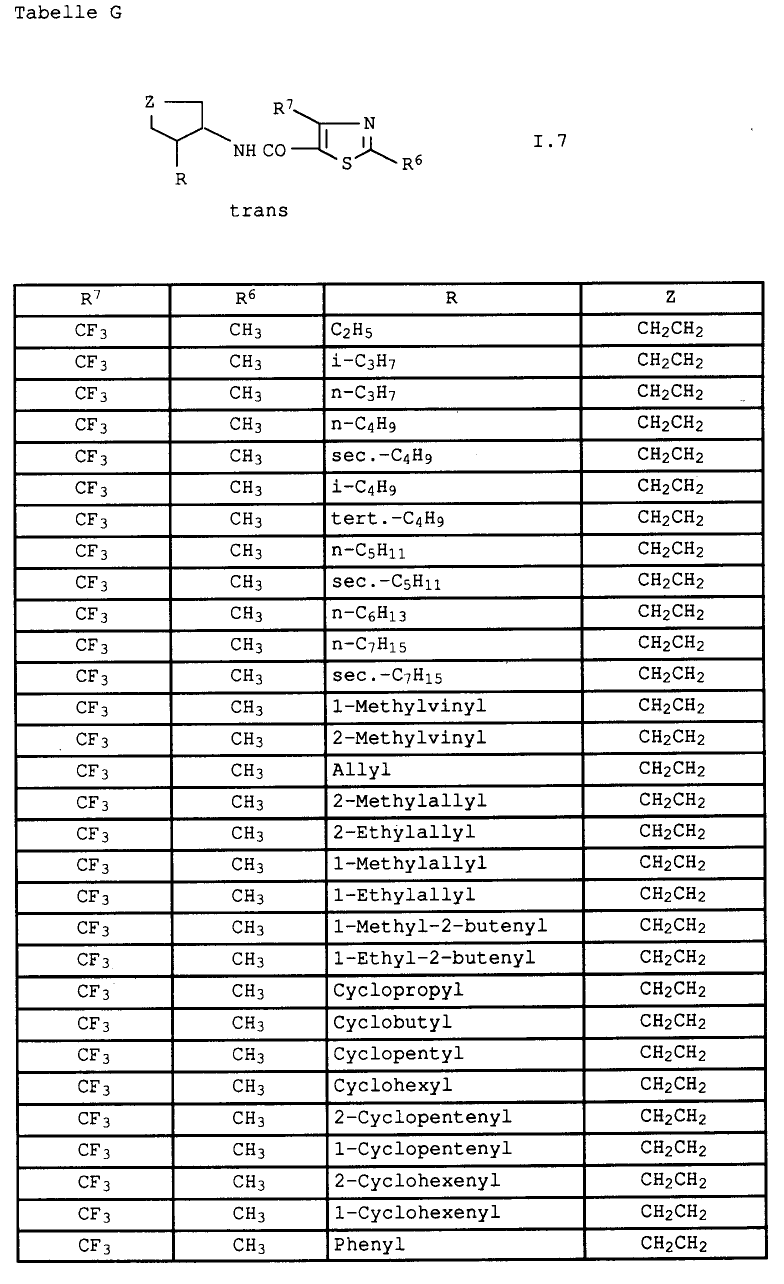

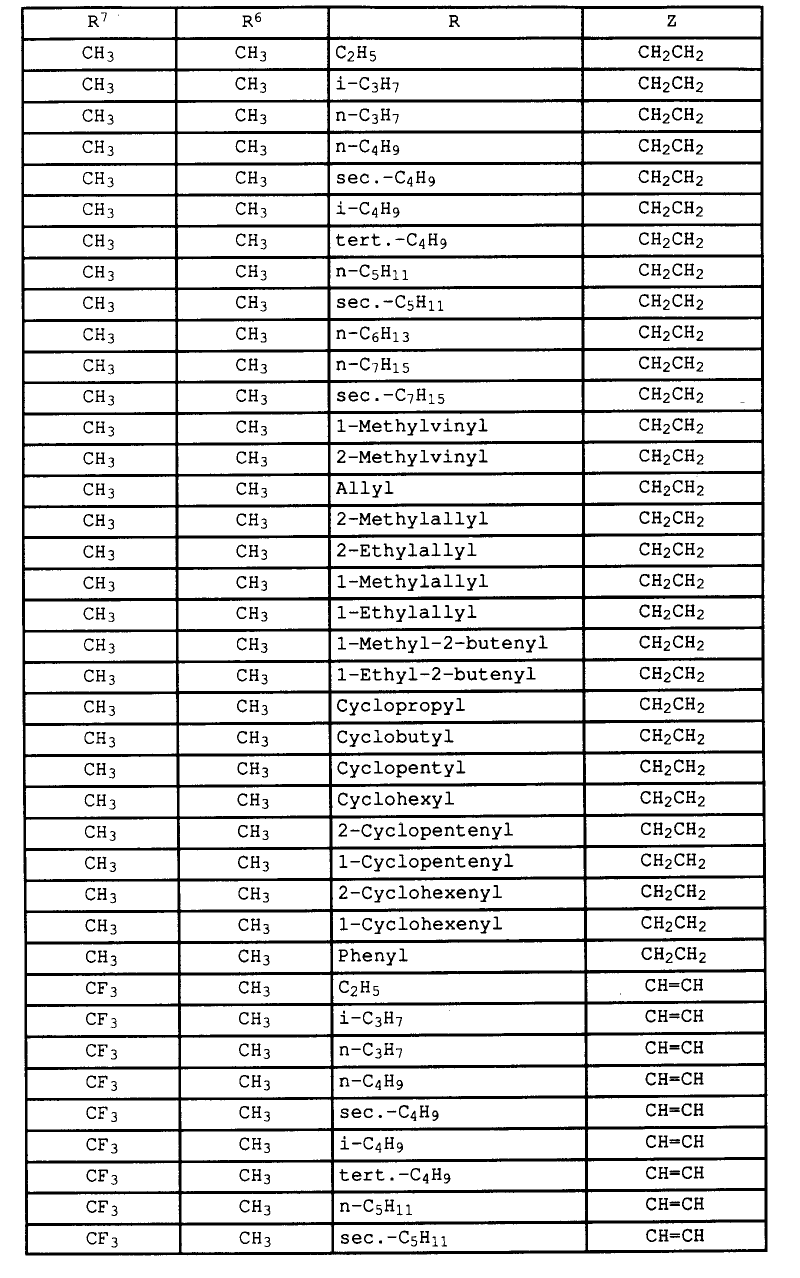

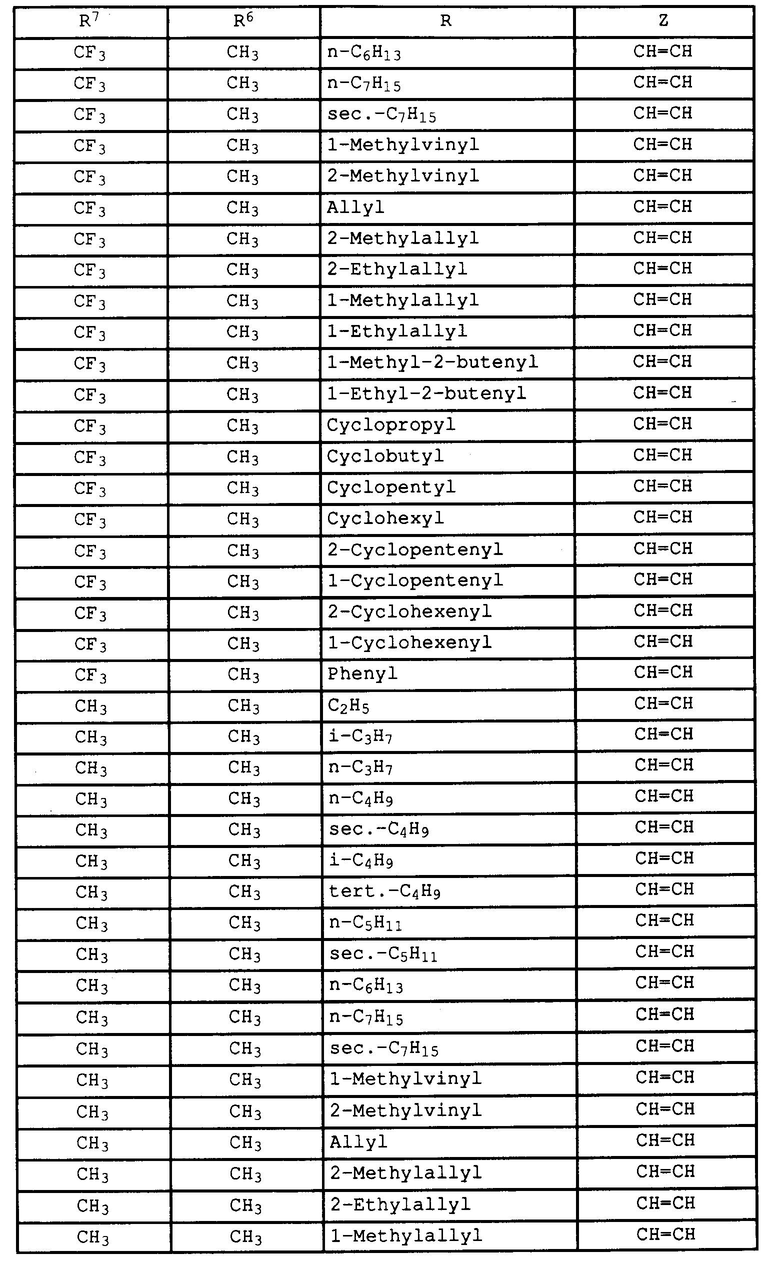

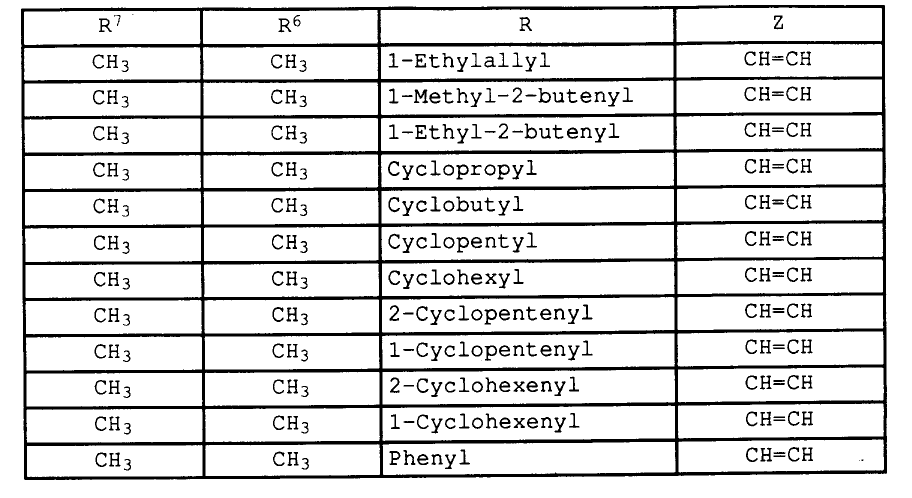

Die neuen Wirkstoffe eignen sich besonders zum Schutz von verschiedenen Materialien gegen den Abbau bzw. die Zerstörung durch Bakterien oder Pilze oder gegen den Befall und Bewuchs durch Mikroorganismen. Materialien, die mit den neuen Wirkstoffen konserviert bzw. mikrozid ausgerüstet werden können, sind beispielsweise Leime und Klebstoffe, Stärkelösungen, Wachsemulsionen, Tonemulsionen, Schlichten, Appreturen, Spinnbäder, Gelatinezubereitungen, Fensterkitt, Fugendichtungsmassen, Kühlschmierstoffe, Bohröle, Treibstoffe, Kunststoffdispersionen, Dispersionsfarben, Textilien, Leder, Rohhäute und Kosmetika. Weiterhin sind die Verbindungen als Schleimbekämpfungsmittel in der Papierindustrie, in Rückkühlwerken und in Luftbefeuchtungsanlagen geeignet.

Des weiteren eignen sich die Verbindungen I zum Schutz folgender Pflanzenarten vor dem Befall durch Mikroorganismen:
Getreide (z.B. Weizen, Gerste, Roggen, Hafer, Reis, Sorhum und Verwandte); Rüben (z.B. Zucker- und Futterrüben); Kern-, Stein- und Beerenobst (z.B. Äpfel, Birnen, Pflaumen, Pfirsiche, Mandeln, Kirschen, Erdbeeren, Himbeeren und Brombeeren); Hülsenfrüchte (z.B. Bohnen, Linsen, Erbsen, Soja); Ölkulturen (z.B. Raps, Senf, Mohn, Oliven, Sonnenblumen, Kokos, Rizinus, Kakao, Erdnüsse); Gurkengewächse (z.B. Kürbis, Gurken, Melonen); Fasergewächse (z.B. Baumwolle, Flachs, Hanf, Jute); Citrusfrüchte (z.B. Orangen, Zitronen, Pampelmusen, Mandarinen); Gemüsesorten (z.B. Spinat, Kopfsalat, Spargel, Kohlarten, Möhren, Zwiebeln, Tomaten, Kartoffeln, Paprika); Lorbeergewächse (z.B. Avocado, Cinnamonum, Kampfer) oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover corps).

Folgende Mikroorganismen lassen sich beispielsweise mit den neuen Verbindungen I bekämpfen:
Straphylococcus aureus, Escherichia coli, Klebsielle pneumoniae, Citrobacter freundii, Proteus vulgaris, Pseudomonas aeruginosa, Desulfovibrio desulfuricans, Streptoverticillium rubrireticuli, Aspergillus niger, Aspergillus versicolor, Penicillium funiculosum, Penicillium expansum, Penicillium glaucum, Paecilomyces variotii, Trichoderma viride, Chaetomium globosum, Aspergillus amstelodami, Phoma pigmentovora, Phoma violacea, Aureobasidium pullulans, Saccharomyces cerevisiae, Alternaria tenuis, Stemphylium macrosporoideum, Cladosporium herbarum, Cladosporium resinae, Candida albicans, Trichophyton mentagrophytes, Geotrichum candidans, Monilia sitophila, Scenedesmus quadricauda, Chlorella vulgaris, Nostoc muscorium, Oscillatoria limosa und Anabaena constricta.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanzen gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser, Trägerstoffe wie natürliche Gesteinsmehle, z.B. Kaoline, Tonerden, Talkum, Kreide und synthetische Gesteinsmehle (z.B. hochdisperse Kieselsäure, Silikate), Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die Formulierungen enthalten im allgemeinen zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR/HPLC/GC-Spektrum) eingesetzt.

Als übliche Anwendungskonzentration wählt man - bezogen auf das Gewicht des zu schützenden Materials - 0,001 bis 5 Gew.-%, bevorzugt 0,01 bis 2 Gew.-% an Wirkstoff; beim Einsatz zur Wasserbehandlung, bei der Erdölförderung, in Bohr- und Schneidölen, Treibstoffen, in Schwimmbädern, Rückkühlwerken, Luftbefeuchtungsanlagen oder in der Papierindustrie sind Wirkstoffmengen von 5 bis 500 ppm ausreichend. Gebrauchsfertige Desinfektionsmittellösungen enthalten z.B. 0,5 bis 10 Gew.-% an Wirkstoff.

Beispiele für solche Zubereitungen sind:
I. eine Lösung aus 90 Gew.-Teilen der Verbindung Nr. 3 und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 5, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.
III. eine wäßrige Dispersion aus 20 Gew.-Teilen der Verbindung Nr. 2, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.
IV. eine wäßrige Dispersion aus 20 Gew.-Teilen der Verbindung Nr. 4, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;
V. eine in einer Hammermühle vermahlene Mischung aus 80 Gew.-Teilen der Verbindung Nr. 1, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;
VI. eine innige Mischung aus 3 Gew.-Teilen der Verbindung Nr. 6 und 97 Gew.-Teilen feinteiligem Kaolin. Dieses Stäubemittel enthält 3 Gew.-% Wirkstoff;
VII. eine innige Mischung aus 30 Gew.-Teilen der Verbindung Nr. 9, 92 Gew.-Teilen pulverförmigem Kieselsäuregel und 8 Gew.-Teilen Paraffinöl, das auf die Oberfläche dieses Kieselsäuregels gesprüht wurde. Diese Aufbereitung gibt dem Wirkstoff eine gute Haftfähigkeit;
VIII. eine stabile wäßrige Dispersion aus 40 Gew.-Teilen der Verbindung Nr. 7, 10 Gew.-Teilen des Natriumsalzes eines Phenosulfonsäure harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 8, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd Kondensates und 68 Gew.-Teilen eines paraffinischen Mineralöls;
X. eine in einer Hammermühle vermahlene Mischung aus 10 Gew.-Teilen der Verbindung Nr. 10, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Wirkstoffe wirken für sich allein als schaumarme Biozide. Eine bedeutende Steigerung der Wirkung dieser Verbindungen enthaltender biozider Zubereitungen wird erzielt, wenn man ihnen noch Tri-C₆- bis C₁₂-alkylmethylammoniumsalze, vorzugsweise in Mengen von 20 bis 40 Gew.-%, bezogen auf das Gewicht der Verbindungen der allgemeinen Formel I, zusetzt.

Die Wirkstoffe können auch mit anderen bekannten Mikrobiziden gemischt werden. In vielen Fällen erhält man dabei einen synergistischen Effekt, d.h. die mikrobizide Wirksamkeit der Mischung ist größer als die der (addierten) Wirksamkeiten der Einzelkomponenten.

Die Zumischung der bekannten Mikrobizide zu den neuen Substanzen kann in einem Gewichtsverhältnis von 1:100 bis 100:1 erfolgen.

### Solche Wirkstoffe sind beispielsweise:

2-(Thiocyanomethylthio)-benzthiazol
1-[2-(2,4-Dichlorphenyl)-2-(2-propenyl-oxy)-ethyl]-1H-imidazol
2,4,5,6-Tetrachlor-isophthalodinitril
Methylenbisthiocyanat
Tributylzinnoxid, -naphthenat, -benzoat, -salicylat
Mercaptobenzthiazol
1,2-Benzisothiazolon und seine Alkalisalze
Alkaliverbindungen des N'-Hydroxy-N-cyclohexyl-diazeniumoxids
2-(Methoxy-carbonylamino)-benzimidazol
2-Methyl-3-oxo-5-chlor-thiazolin-3-on
Trihydroxymethyl-nitro-methan
Glutardialdehyd
Chloracetamid
Polyhexamethylenbisguanide
5-Chlor-2-methyl-4-isothiazolin-3-on + Magnesiumsalze
3,5-Dimethyltetrahydro-1,3,5-2H-thiadiazin-2-thion
Hexahydrotriazin
N,N-Methylolchloracetamid
2-n-Octyl-4-isothiazol-in-3-on
Oxazolidine
Bisoxazolidine
2,5-Dihydro-2,5-dialkoxy-2,5-dialkylfurane
Diethyl-dodecyl-benzyl-ammoniumchlorid
Dimethyl-octadecyl-dimethylbenzyl-ammoniumchlorid
Dimethyl-didecyl-ammoniumchlorid
Dimethyl-didodecyl-ammoniumchlorid
Trimethyl-tetradecylammoniumchlorid
Benzyl-dimethyl-alkyl-(C₁₂-C₁₈)-ammoniumchlorid
Dichlorbenzyl-dimethyl-dodecyl-ammoniumchlorid
Cetylpyridiniumchlorid
Cetylpyridiniumbromid
Cetyl-trimethyl-ammoniumchlorid
Laurylpyridiniumchlorid
Laurylpyridiniumbisulfat
Benzyl-dodecyl-di(beta-oxyethyl)-ammoniumchlorid
Dodecylbenzyl-trimethyl-ammoniumchlorid
n-Alkyl-dimethyl-benzyl-ammoniumchlorid
(Alkylrest: 40 % C₁₂, 50 % C₁₄, 10 % C₁₆)
Lauryl-dimethyl-ethyl-ammoniumethylsulfat
n-Alkyl-dimethyl-(1-naphthylmethyl)-ammoniumchlorid
(Alkylrest: 98 % C₁₂, 2 % C₁₄)
Cetyldimethylbenzylammoniumchlorid
Lauryldimethylbenzylammoniumchlorid

### Weitere mögliche Mischungspartner sind beispielsweise:

1,3-Dimethylol-5,5-dimethylhydantoin
Dimethylolharnstoff
Tetramethylolacetylendiharnstoff
Dimethylolglyoxalmonourein
Hexamethylentetramin
Glyoxal
Glutardialdehyd
N-Methylol-chloracetamid
1-(Hydroxymethyl)-5,5-dimethyl-hydantoin
1,3-Bis-(hydroxymethyl)-5,5-dimethylhydantoin
Imidazolidinylharnstoff
1-(3-Chlorallyl)-3,5,7-triaza-1-azonia-adamantan-chlorid
1,3-Bis-(β-ethylhexyl)-5-methyl-5-amino-hexahydropyrimidin
1,3,5-Tris-(hydroxyethyl)-1,3,5-hexahydrotriazin
1,2-Dibrom-2,4-dicyanobutan
5-Brom-5-nitro-1,3-dioxan
2-Brom-2-nitropropandiol
1,1'-Hexamethylen-bis-[5-(4-chlorphenyl)-biguanid]
4,4-Diaminodiphenoxypropan
2-Brom-2-nitro-propan-1,3-diol
Sorbinsäure und ihre Salze
p-Hydroxybenzoesäure und ihre Ester und Salze
Zink-2-pyridinethiol-N-oxid
2-[(Hydroxylmethyl)amino]-ethanol
Dithio-2,2'-bis(benzmethyl-amid)
5-Chlor-2-(2,4-dichlorphenoxy)-phenol
Thio-bis-(4-chlorphenol)
o-Phenyl-phenol
Chlormethyl-dijodmethylsulfon
p-Chlorphenyl-3-jodpropargyl-formal

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I genutzt. Die so erhaltenen Verbindungen sind in den anschließenden Tabellen mit physikalischen Daten aufgeführt.

### 1. N-[2-(1-Methylpropyl)-cyclohexyl]-2-methyl-4-trifluormethyl-thiazol-5-carbonsäureamid

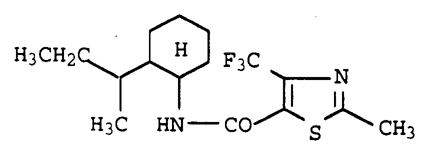

Zu einer Lösung 2,3 g trans-2-sec.-Butylcyclohexylamin und 1,5 g Triethylamin in 15 ml Tetrahydrofuran werden bei 0°C 3,4 g 2-Methyl-4-trifluormethylthiazol-5-carbonsäurechlorid zugetropft und 2 Stunden bei 25°C nachgerührt. Nach Verdünnen des Ansatzes mit 300 ml Wasser und zweimaligem Extrahieren mit tert.-Butylmethylether, Trocknen und Verdampfen des Lösungsmittels und Anteigen des Rückstands mit wenig n-Pentan isoliert man 2,3 g 2-Methyl-4-trifluormethyl-thiazol-5-carbonsäure-trans-2-sec-butylcyclohexylamid vom Fp. 112 - 113°C.

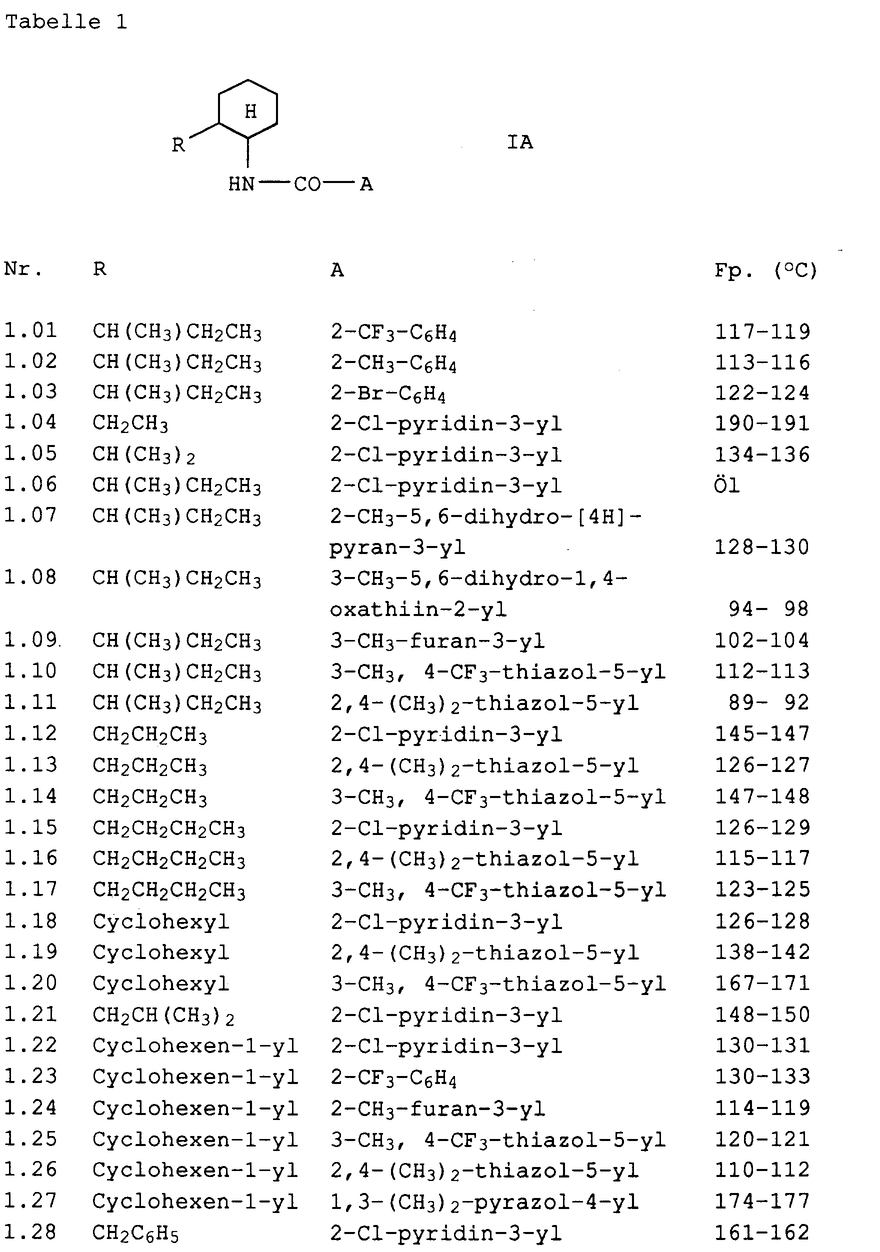

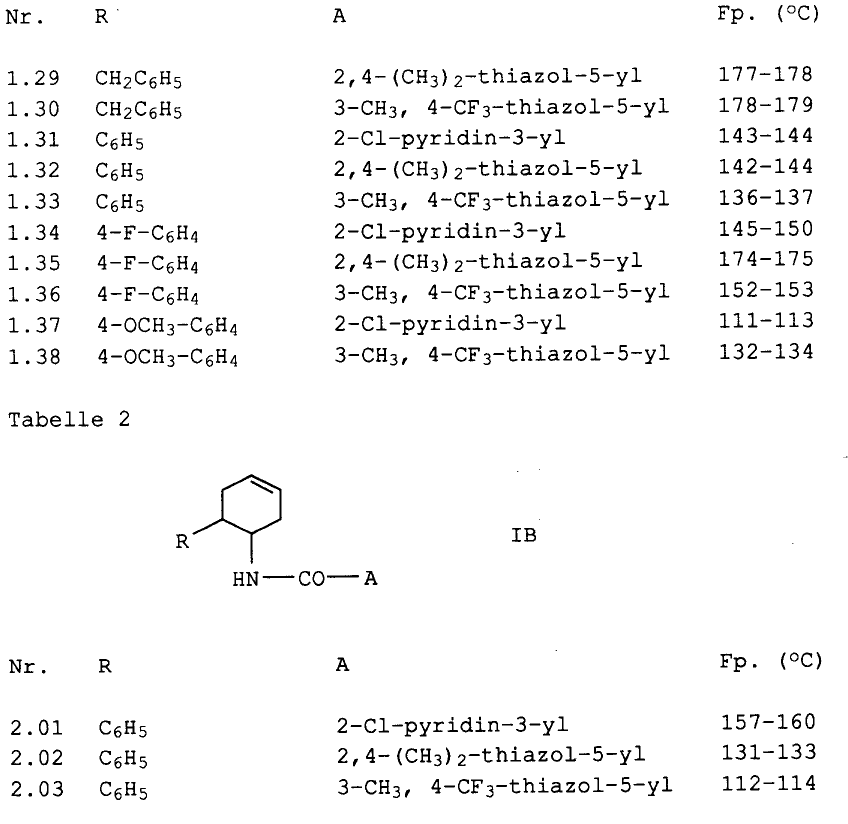

### Beispiele zur biologischen Wirkung:

### Wirksamkeit gegen Botrytis cinerea

Scheiben von grünen Paprikaschoten wurden mit einer wäßrigen Suspension [80 % Wirkstoff / 20 % Emulgator in der Trockenmasse] des Wirkstoffs tropfnaß gespritzt. Nach dem Abtrocknen des Spritzbelags wurden die Scheiben mit einer Sporensuspension [1,7·10⁶ Sporen pro ml; 2 % Biomalz; Wasser] des Pilzes Botrytis cinerea besprüht und anschließend 4 Tage bei 18°C und hoher Luftfeuchtigkeit aufbewahrt.

Nach dieser Zeit wiesen die nicht mit Wirkstoff vorbehandelten Kontrollen einen Pilzbefall von 90 % auf, während die mit jeweils 500 ppm der Verbindungen Nr. 5, 6, 7, 8, 9, 10, 11, 12, 13, 15, 16, 19, 24, 25, 26, 27 und 32 behandelten Paprika-Scheiben maximal zu 15 % befallen waren.

Bei einer Aufwandmenge von 1000 ppm der Verbindungen Nr. 4, 5 und 6 wiesen die behandelten Paprika-Scheiben maximal 15 % Befall auf, während Paprika-Scheiben, die mit 1000 ppm N-(2-Methylcyclohexyl)-2-chlornicotinsäureamid behandelt waren, einen Befall von 40 % aufwiesen.

## Patentansprüche

1. N-Cyclohex(en)ylcarbonsäureamide der Formel I 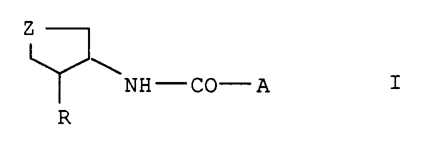 in der die Substituenten die folgende Bedeutung haben:
R C₂-C₁₂-Alkyl, C₂-C₁₂-Alkoxy, C₃-C₁₂-Alkenyl, C₃-C₁₂-Alkenyloxy, C₃-C₆-Alkinyl, C₃-C₆-Alkinyloxy, wobei diese Gruppen partiell oder vollständig halogeniert sein können; C₃-C₇-Cycloalkyl, C₄-C₇-Cycloalkenyl, C₃-C₇-Cycloalkyloxy oder C₄-C₇-Cycloalkenyloxy, wobei diese Ringe ein bis drei C₁-C₄-Alkylgruppen tragen können; Phenyl oder Benzyl, wobei die Phenylringe jeweils ein bis fünf Halogenatome und/oder ein bis drei der folgenden Reste tragen können: C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogenalkylthio;
Z CH₂CH₂ oder CH=CH;
A ein cyclischer Rest aus der Gruppe der Formeln A1 bis A7 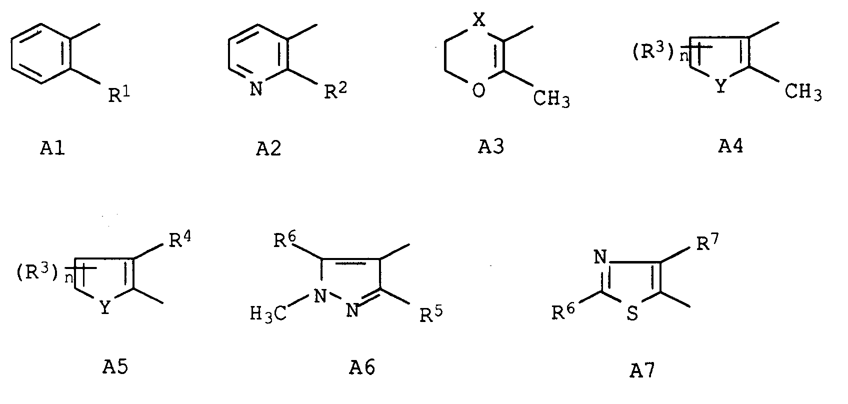 in denen die Substituenten die folgende Bedeutung haben:
X -CH₂-, -S-, -SO- oder -SO₂-;
Y -O- oder -S-;
R¹, R², R⁴, R⁵ und R⁷ Halogen, C₁-C₄-Alkyl oder C₁-C₄-Halogenalkyl;
R³ und R⁶ Wasserstoff, Halogen oder C₁-C₄-Alkyl;
n 1 oder 2, wobei die Reste R³ verschieden sein können, wenn der Wert von n 2 beträgt.

2. N-Cyclohex(en)ylcarbonsäureamide der Formel I, gemäß Anspruch 1, in der R die in Anspruch 1 gegebene Bedeutung hat und A für einen cyclischen Rest aus der Gruppe der Formeln A1 bis A7 steht, in denen X und Y die in Anspruch 1 gegebene Bedeutung und die Substituenten die folgende Bedeutung haben:
R¹ Halogen, Methyl oder C₁-Halogenalkyl;
R² Halogen oder C₁-Halogenalkyl;
R³ Wasserstoff oder Methyl;
n 1 oder 2, wobei die Reste R³ verschieden sein können, wenn der Wert von n 2 beträgt;
R⁴ Halogen, oder Methyl;
R⁵ Methyl oder C₁-Halogenalkyl;
R⁶ Wasserstoff, Halogen oder Methyl;
R⁷ Halogen, Methyl oder C₁-Halogenalkyl.

3. N-Cyclohex(en)ylcarbonsäureamide der Formel I, gemäß Anspruch 1, in der R die in Anspruch 1 gegebene Bedeutung hat und A für einen cyclischen Rest aus der Gruppe der Formeln A1 bis A7 steht, in denen X und Y die in Anspruch 1 gegebene Bedeutung und die Substituenten die folgende Bedeutung haben:
R¹ Chlor, Brom, Jod, Methyl oder Trifluormethyl;
R² Chlor oder Trifluormethyl;
R³ Wasserstoff oder Methyl;
n 1 oder 2, wobei die Reste R³ verschieden sein können, wenn der Wert von n 2 beträgt;
R⁴ Chlor oder Methyl;
R⁵ Methyl, Difluormethyl oder Trifluormethyl;
R⁶ Wasserstoff, Chlor oder Methyl;
R⁷ Chlor, Methyl oder Trifluormethyl.

4. N-Cyclohex(en)ylcarbonsäureamide der Formel I, gemäß Anspruch 1, in denen die Reste R und NHCOA trans zueinander stehen.

5. Verfahren zur Herstellung der Verbindungen I gemäß Anspruch 1, dadurch gekennzeichnet, daß man ein Carbonsäurehalogenid der Formel II
Hal-CO-A II
in der Hal für ein Halogenatom steht, in an sich bekannter Weise in Gegenwart einer Base mit einem Cyclohexylamin der Formel III 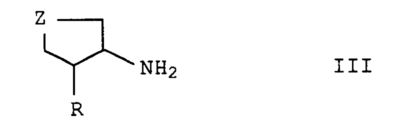 umsetzt.

6. Mittel zur Bekämpfung von Schadpilzen, enthaltend eine fungizide Menge einer Verbindung der Formel I gemäß Anspruch 1, 2, 3 oder 4 und inerte Zusatzstoffe.

7. Verfahren zur Bekämpfung von Schadpilzen, dadurch gekennzeichnet, daß man die Schadpilze, ihren Lebensraum und/oder die von Schadpilzen freizuhaltenden Pflanzen oder Materialien mit einer fungizid wirksamen Menge einer Verbindung der Formel I gemäß Anspruch 1, 2, 3 oder 4 behandelt.

8. Verwendung der Verbindungen I gemäß Anspruch 1, 2, 3 oder 4 zur Bekämpfung von Schadpilzen.

9. Verwendung der Verbindungen I gemäß Anspruch 1, 2, 3 oder 4 zur Bekämpfung von Botrytis.
